# EUROPEAN PATENT SPECIFICATION

(11) **EP 2 617 072 B1**
(45) Date of publication and mention of the grant of the patent: **06.05.2020**
(21) Application number: 11825739.3
(22) Date of filing: 12.09.2011
(51) Int. Cl.: H01L 35/32, H01C 13/02, H01C 1/16, H01C 1/12, H01C 7/00

(54) **DISTRIBUTED THERMOELECTRIC STRING AND INSULATING PANEL AND APPLICATIONS FOR LOCAL HEATING, LOCAL COOLING, AND POWER GENERATION FROM HEAT**
VERTEILTES WÄRMESTROMBAND SOWIE DÄMMPLATTE UND ANWENDUNGEN FÜR LOKALE ERWÄRMUNG, LOKALE KÜHLUNG UND STROMERZEUGUNG AUS WÄRME
GUIRLANDE THERMOÉLECTRIQUE DISTRIBUÉE ET PANNEAU ISOLANT, ET LEURS APPLICATIONS POUR LE CHAUFFAGE LOCAL, LE REFROIDISSEMENT LOCAL, ET LA PRODUCTION D'ÉNERGIE À PARTIR DE CHALEUR

(30) Priority: 06.07.2011 US 201161504784 P; 04.05.2011 US 201113101015; 31.03.2011 US 201161470039 P; 17.01.2011 US 201161433489 P; 26.11.2010 US 417380 P; 13.09.2010 US 403217 P
(43) Date of publication of application: 24.07.2013
(73) Proprietor: Tempronics, Inc., Tucson, AZ 85716 (US)
(72) Inventor: MAKANSI, Tarek, Tucson Arizona 85750 (US); BERMAN, Michael, Tucson Arizona 85750 (US); WOOD, Steve, Tucson Arizona 85750 (US); FRANKLIN, John, Tucson Arizona 85750 (US); EVERS, Mark, N., Tucson Arizona 85750 (US)
(74) Representative: Prinz & Partner mbB
(86) International application number: PCT/US2011/051227
(87) International publication number: WO 2012/037031

(56) References cited:
- JP-A- 2003 209 297
- US-A- 2 858 350
- US-A- 3 196 524
- US-A- 6 129 990
- US-A1- 2007 112 390
- US-A1- 2008 029 146
- US-A1- 2009 025 774
- US-A1- 2010 107 657
- US-B2- 6 863 981
- LAUTERBACH C ET AL: "Smart Clothes Self Powered by Body Heat", INTERNET CITATION, 2002, XP002471340, Retrieved from the Internet: URL:http://www.future-shape.com/publicatio ns.html [retrieved on 2008-03-03]

## Description

Thermoelectric modules typically contain densely packed elements spaced apart by 1-3 mm. Typically, up to 256 such elements may be connected in an array that is 2x2 inches (5.08 x 5.08 cm) in area. When these modules are deployed, large and heavy heat sinks and powerful fans are required to dissipate or absorb the heat on each side. The reasons for these dense prior art configurations are well-founded: small elements with low resistance allow larger current I to flow before the resistive heat (12R) generated destroys the thermoelectric cooling (pl1 where p = Peltier coefficient). The use of short elements for maximum cooling capacity results in the hot and cold side circuit boards being close together. This proximity results in the high density.

To achieve a low density packing of thermoelectric elements, one could space out these elements on the boards laterally, but then the backflow of heat conducted and radiated through the air between the elements limits the overall performance. Some designs require evacuating the module interior to reduce heat backflow due to air conduction, but vacuum cavities require expensive materials and are prone to leaks. Vacuum materials (like glass and Kovar™) are also hard and easily broken when thin enough to limit their own backflow of heat. Broken glass can lead to safety issues when these modules are used in seat cushions, automobiles, and other environments.

Another problem in spreading out thermoelectric elements is that the rigid connection of elements over large distances causes them to rupture due to sheer stress upon thermal expansion of the hot side relative to the cold side. To solve this problem, other designs have been proposed that use a flexible plastic such as polyimide for the circuit boards, but these materials are too porous to maintain a vacuum. Another disadvantage of the prior art design of thermoelectric modules is that the high density of heat moved to the hot side results in a temperature gradient through the heat sink, and this temperature delta subtracts from the overal I cooling that the module can achieve, in particular, traditional thermoelectric products are not able to reach true refrigeration temperature because of this temperature gradient.

Finally, because prior art thermoelectric modules are placed in a solder reflow oven during assembly, only high-temperature materials may be used. Unfortunately, many desired uses of cooling and heating involve close or direct contact with the human body, for which soft materials, such as cushions, cloths, and flexible foam are preferred, but these materials cannot withstand the high temperatures of a solder reflow oven.

Thermoelectric devices can be as efficient, or even more efficient, than vapor compression cooling systems when the temperature delta is 10 degrees C or less. For this reason, a strong desire exists to deploy thermoelectric technology for local heating and cooling of occupied spaces and thereby red uce the overall energy consumption needed for personal comfort. The total energy savings of the central A/C or heating system plus the local thermoelectric systems can be 30% or more for such a combination, but the unwieldy implementation of prior-art thermoelectric modules inhibits their use for this purpose.

Most thermoelectric and compressor-based cooling systems today are configured as forced air systems. In order to cool the room to a comfortable 75F, the forced air needs to be 55F as it exits the vent. The difference between the 55F cold side temperature and the outside temperature of 80F to 1 10F means that the delta temperature across a thermoelectric module in a forced-air configuration is so large that its heat-back flow conduction makes its overall efficiency very low. However, if a distributed thermoelectric implementation is used, as disclosed here, the cold side can be in contact with the human body, or in close enough proximity such that the cold side seen by the thermoelectric elements are close to the ideal skin temperature of 86-9 IF, hence reducing the temperature delta at the thermoelectric device to a level that makes its efficiency comparable to that of a compressor-based system.

Individuals who sit or lie down for long periods of time experience discomfort from trapped heat between the skin and the contact surface. This trapped body heat leads to unproductive perspiration which accumulates and causes a soggy, sticky feeling. In extreme cases, the moisture weakens the skin and the tissue causing pressure ulcers and sores. Although these skin disorders are fundamentally caused by pressure closing off blood flow to tissues, temperature is also a factor in their formation and severity (see "Skin Cooling Surfaces: Estimating the Importance of Limiting Skin Temperature", by Charles Lachenbruch, Ostomy Wound Management Feb 2005). A distributed thermoelectric implementation can be very effective in eliminating the discomfort and reducing or preventing the disorders caused by trapped heat in sitting and lying down positions.

In one example, we show how a string of thermoelectric elements connected by conductors in accordance with the present invention (see below) can be used to produce a heated or cooled mattress surface. The resulting mattress uses contact between the expanded conductor and the skin or clothing to remove trapped heat, which is not only more efficient as mentioned earlier, but also is responds much faster than prior art thermoelectric systems that employ a working fluid like water or air. In these prior art systems, the entire volume of the water or air must have its temperature increased or decreased before the user feels a change. For the present invention, the user feels a change as soon as the expanded conductor changes temperature, which can occur in seconds.

Hence, the need exists for a variety of insulating panels to be safely and comfortably improved with thermoelectric capability, such as seat cushions, mattresses, pillows, blankets, cei ling tiles, office/residence walls or partitions, under-desk panels, electronic enclosures, building walls, solar panels, refrigerator walls, freezer walls within refrigerators, or crisper walls within refrigerators.

Devices that generate electricity from renewable sources all have limitations. The ideal power generation technology supplies power 24 hours per day, is low cost, and uses only energy from renewable sources, such as wind, tidal and wave, sunlight, or geothermal pools. The two most common forms of utility-scale renewable power generation are wind turbines and photovoltaic systems.

Photovoltaic (PV) technology has the following limitations: (1) high cost, (2) generates power only when the sun is shining brightly which is less than 33% of the time, (3) introduces transients into the electrical grid when clouds suddenly block the sun, and (4) low efficiency without concentration or dangerous temperatures and light levels with concentration.

Wind turbines have the following limitations: (1) relatively high cost, (2) generates power only when the wind is blowing which is less than 33% of the time on average, (3) introduces transients into the electrical grid when the wind suddenly stops or changes direction, (4) requires very tall and visually unacceptable structures, (5) generates noise, (6) has a random peak capacity time during the day that rarely matches the peak demand time, and (7) has very low land usage at about 4 Kwatts per acre.

Both PV and wind turbines may be supplemented with large batteries to store energy for periods of time when the renewable source is not available, but such storage is very expensive at about $1000 per Kwatt hour. When combined with battery storage to achieve 100% renewable generation, the cost for a renewable PV or wind turbine plant is around $20 per watt, vs. about $10 per watt for a fossil fuel pant including 10 years of fuel costs.

Tidal and wave energy installations require high capital startup costs, and like wind turbines, suffer from variable output and may be visually unacceptable structures if erected near shorelines.

Hence, the need exists for a low-cost electrical power generation capability that can supply power 24 hours per day, 7 days per week, and 365 days per year and only tap renewable energy sources.

US 2008/0029146 A1 discloses a thermoelectric structure comprising a network of wires oriented substantially in weft direction of a woven structure. Conduction wires are interwoven to form hot and cold junctions distributed in the top and bottom planes of the structure. Dielectric wires are interwoven with the first and second conducting wires so as to keep the top and bottom planes at a distance.

The article "Smart Clothes Self Powered by Body Heat", Lauterbach, C. et al., Internet Citation, 2002, XP 002471340, discloses how thermoelectric devices may be used in clothing articles. A schematic illustration is given of a thermo generator integrated into a fabric cover where conducting wires run through the fabric for interconnecting the successive thermoelectric elements.

Broadly speaking, this invention makes possible thermoelectric capability for a variety of panel materials and enables local/personal heating and cooling that reduces overall energy consumption. According to the present invention a string of thermoelectric elements connected to one another by stranded wire conductors and a thermoelectric panel comprising such thermoelectric elements woven in and out of holes in a panel is provided as defined in claims 1 and 3 respectively. A thermoelectric device comprising a plurality of such thermoelectric panels is provided as defined in claim 4.

According to the present invention, a method of forming a thermoelectric panel is also provided as defined in claim 6. A thermoelectric device comprising the thermoelectric panel for generating electricity is provided as defined in claim 7. An apparatus for storing energy from the sun and generating electricity making use of at least one string of thermoelectric elements as defined in claim 1 is also provided as set out in claim 8. Moreover, uses of the string of thermoelectric elements and the thermoelectric panel are also provided according to the present invention as defined in claims 10, 11, 12 and 13.

One embodiment provides a thermoelectric string that can be woven or inserted into a variety of such panels, including soft and low-temperature panels. Another embodiment also eliminates the need for a large, bulky, heavy, and expensive heat sinks and fans to dissipate heating and cooling. A further embodiment combines hardware that moves electrical current with hardware that dissipates thermal energy, thereby saving cost over embodiments such as US 3, 196,524. Another embodiment provides a common set of hardware to provide low thermal back flow near the thermoelectric elements and simultaneously provides high thermal conduction to ambient air away from the elements. In one embodiment this invention provides a thermoelectric string that can be routed through small holes in the panel to minimize thermal leakage. In another embodiment this invention eliminates the need for vacuum enclosures such as US 3,225,549 of highly- distributed thermoelectric elements and also eliminate the need for wicking fluids such as US 2010/0107657. In a particularly preferred embodiment this invention provides cooling capability and electricity generation for pennies per watt in manufacturing cost. In some embodiments this invention reduces the delta temperature required across the thermoelectric elements to a level that the overall cooling efficiency can be comparable to that of a vapor compression system. In other embodiments, this invention reduces or eliminates discomfort and disorders from trapped heat between human or animal skin and surfaces.

Features and advantages of the present invention will be seen from the following detailed description taken into conjunction with the accompanying drawings wherein like numerals depict like parts, and wherein:
FIG. I a shows a string of thermoelectric elements connected by lengths of braided wire with a flat (pellet) strain reliefs;
FIG. Ib shows a string of thermoelectric elements connected by lengths of braided wire with tubular strain reliefs
FIGs. 2a and 2b illustrate a method of assembling the thermoelectric elements on strain reliefs using a standard circuit board manufacturing process; FIG. 3a illustrates how the braid of FIG. 1a, with pellets, is woven on alternating sides of an insulating panel;
FIG. 3b illustrates how the braid of FIG. 1b, with therm o-tunneling tubes, is woven on to alternating sides of an insulating panel;
FIG. 3c illustrates how the braid of FIG. 1a, with pellets, is woven on to one side of an insulating panel;
FIG. 3d illustrates how the braid of FIG. 1b, with thermo-tunnelling tubes, is woven on one side of an insulating panel.
FIG. 4a illustrates how multiple layers of panels shown in FIG. 3a can be cascaded in order to more efficiently achieve a high temperature difference;
FIG. 4b illustrates how multiple channels of the panels of FIG. 3c can be cascaded in order to more efficiently achieve a high temperature difference;
FIG. 5 is a top plan view of a panel illustrating various examples of how multiple metal materials can serve as expandable heat sinks or heat absorbers;
FIGs. 6a-6i illustrate various expandable metals which advantageously may be employed in the present invention including un-oriented copper mesh (FIG. 6a);
   oriented copper mesh (FIG. 6b); flat copper braid (FIG. 6c): tubular copper braid (FIG 6d); copper rope (FIG. 6e); copper tinsel with wire center (FIG. 6f); oriented stranded copper (FIG. 6g); copper foam (FIG. 6h); and un-oriented stranded copper (FIG. 6i).
FIGS. 7a-c illustrated a thermoelectric cooler made in accordance with the present invention, and FIG. 7d plots time versus temperature comparing a
   thermoelectric cooler of the present invention with a prior art commercial cooler;
FIG. 8 illustrates, without limitation, many of the applications for the panel of FIG. 3 or FIG. 4 for heating and cooling functionality;
FIG. 9 illustrates one application for the panel of FIG. 3 or FIG. 4 for generating electricity from a heat storage medium heated by the sun;
FIG. 10 shows the panel of FIG. 3 or FIG. 4 providing heating and cooling for the surface of a spring mattress;
FIGs. 11a and 1 ib show the same panel providing heating and cooling for the surface of air mattresses;
FIGs, 12a and 12b show the same panel providing heating and cooling for thick foam mattresses;
FIG. 13a is a perspective view, from the side showing a foam mattress made with thermoelectric panels of FIG. 12a;
FIG. 13b is a perspective view from the end of the mattress of FIG. 13a;
FIG. 13c shows the mattress of FIG. 13a with the thermoelectric panel removed;
FIG. 14 shows a picture of a heated and cooled electric blanket built as described in FIG. 8;
FIGs. 15a and 15b show integration of a thermoelectric panel of the present invention into a mesh-style office chair, FIG. 15a shows an expanded thermoelectric string, and FIG. 15b shows a solid thermoelectric string in accordance with the present invention.
FIGs. 16a- 16c illustrate incorporation of a thermoelectric panel as shown in FIG. 8 and FIGs.15a/l 15b in a chair;

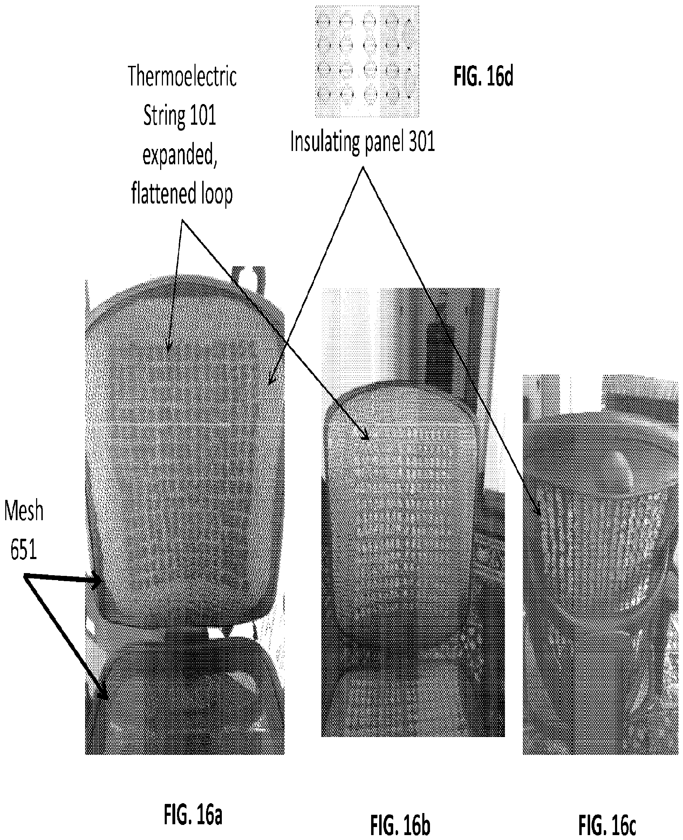

FIG. 16a shows the thermoelectric panel mounted behind the chair mesh;
FIG. 16b shows the thermoelectric string with portion of the string in front of the mesh;
FIG. 16c shows the thermoelectric string mounted on the back of the chair; FIG. 16d shows a thermoelectric panel; and

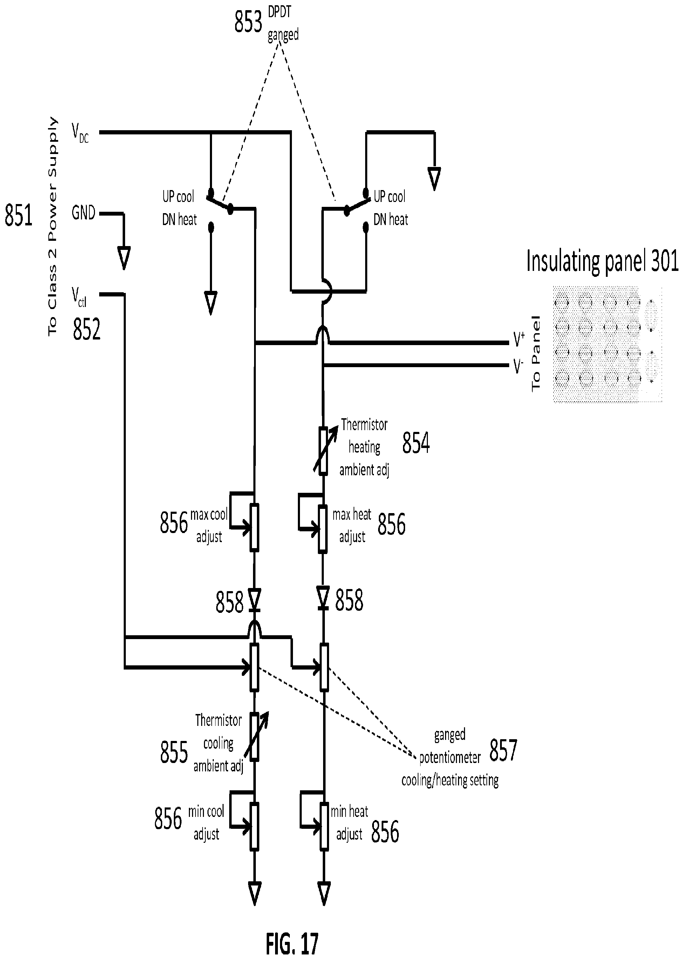

FIG. 17 shows an electronics circuit schematic diagram for a thermoelectric panel made in accordance with the present invention that includes a variable amount of heating and cooling.

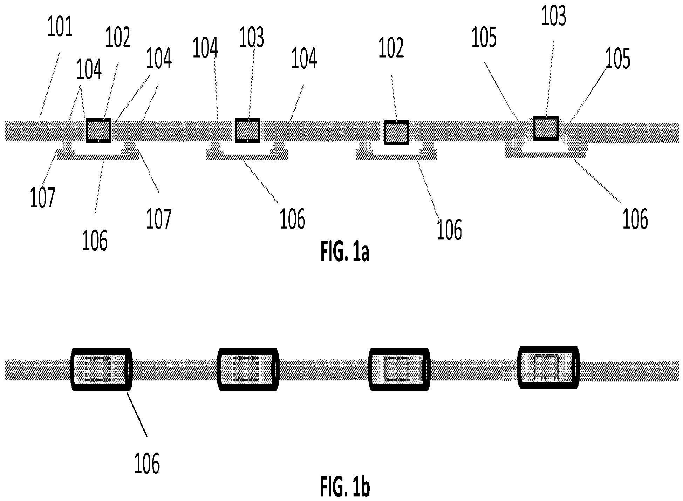

A preferred embodiment of this invention includes a string containing alternating P-type 102 and N-Type 103 thermoelectric elements connected by lengths of braided or stranded wire 101 as shown in FIGs. 1 a and 1 b. The thermoelectric elements preferably comprise metals, although non-metallic conductors such as graphite and carbon may be used. In one embodiment, the alternating elements can be small crystals of, e.g. Bismuth Telluride (N-type) 103 and, e.g. Antimony Bismuth Telluride (P-type) 102, possibly plated with, e.g. Nickel and/or Tin on the ends to facilitate solder connections 104 or 105, or can be small thermo-tunneling vacuum tubes. Because the thermoelectric elements or tubes may be fragile, a "strain relief, made of a stiff material 106 like FR4 combined with copper 107 and solder 104 or 105 bonds prevents a pulling force on the wire from breaking the elements or vacuum tubes. The aggregate diameter of the stranded or braided wire is designed to carry the desired electrical current with minimal resistance. In FIG. 1a, the strain relief is flat, facilitating its manufacture using standard circuit board manufacturing processes. In FIG. 1b, the strain relief is a tubular sleeve that is positioned over the elements 102 or 103 and over sufficient lengths of the wire 101. The design of FIG. 1b is beneficial if the thermoelectric element must be sealed and in order to save the cost and complexity of the separate circuit board manufacturing steps. Without limitation, the tubular strain relief 106 in FIG. 1b may be a woven fiberglass sleeve threaded over the thermoelectric element combined with an epoxy or glue. Once hardened, the combination of epoxy and the woven fiberglass is very stiff, as these same materials are used to produce the FR-4 circuit boards of FIG. 1a.

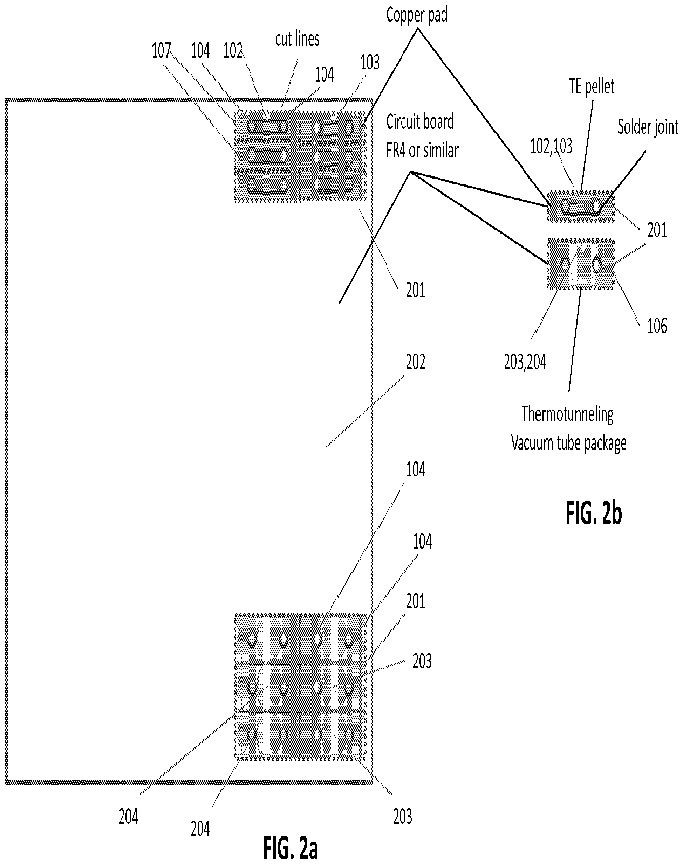

FIGs. 2a and 2b show how subassemblies of this thermoelectric string might be fabricated using standard circuit board assembly techniques and machinery. A large F 4 circuit board 202 is patterned with the copper pads 107 of the strain reliefs 106 of FIG. I a. A packed arrangement is used to assemble the pellets 102 and 103 or tubes 203 and 204 onto the board. An assembly robot can place the thermoelectric elements or tubes and place solder paste 104 at the appropriate joints. The whole assembly is run through an oven to flow the solder and then cooled to harden the solder joints. Once assembly is completed, the strain relief assemblies are cut out along the cut lines 201 to leave the thermoelectric elements mounted on the strain relief 106.

The lower portion of FIG. 2a shows how the invention can also apply to the latest advanced thermo-tunneling devices. Such devices are more efficient, but require packaging in a vacuum tube. These small vacuum tubes can substitute for the thermoelectric elements 102 and 103 of FIGs. 1a and 1b and can also benefit greatly from the strain reliefs 106 of FIGs. 1 a and 1b and 2a and 2b. Since a useful vacuum package must have a thin glass wall to minimize thermal conduction, it will also likely be very fragile.

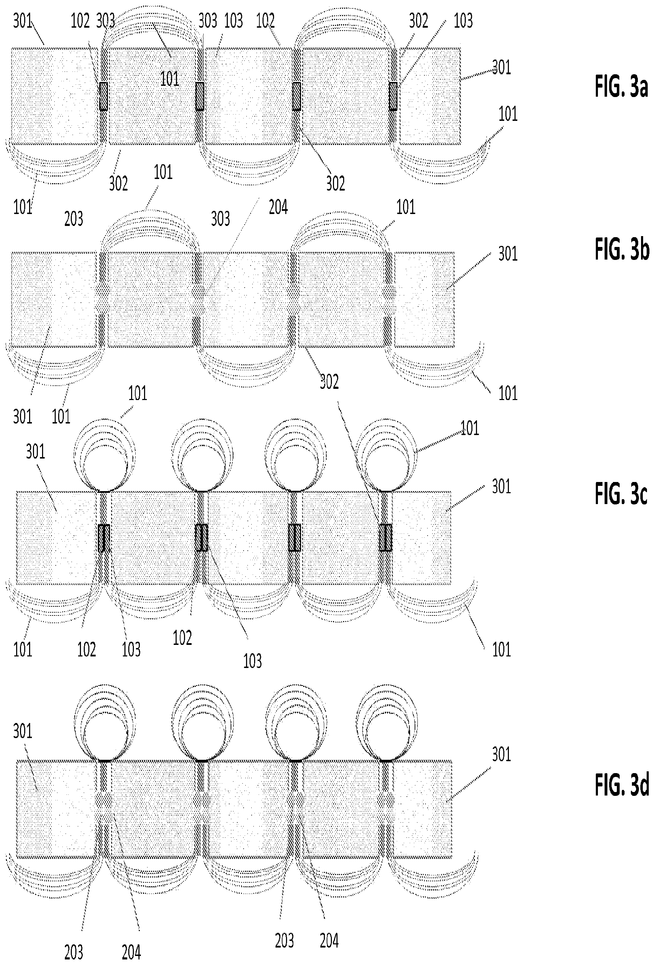

The thermoelectric elements of FIGs. 1a and 1b alternate between N-type 103 and P-type 102 in order to move heat in the same direction while the current flows back and forth along the string woven into a panel 301 as shown in FIG. 3. One purpose of compacting the wire strands in the string of FIGs. 1a and 1b is to be able to route the string through small-diameter holes 302 in the panel. The hole diameter should be small to minimize thermal leakage that compromise the insulating capability of the panel material. Another purpose of compacting the wires near the elements is to minimize the area for heat to backflow from the hot side of the element to the cold side of the element. The string may be woven into the panel 301 in an alternating fashion as illustrated in FIG. 3a and FIG. 3b. Or, the N-type and P-type elements may be paired together to allow the string to be pushed though the holes 302 from one side as illustrated in FIG. 3c and FIG. 3d. The single sided approach in FIG. 3c and 3d facilitates manufacture of the panel from one side rather than having to work with both sides as in FIG. 3a and 3b.

Another embodiment is when the compacted portions 303 of the string within the panel holes of FIGs. 3a and 3b are replaced with solid cylinders made of copper or similar metal and these cylinders are attached to the thermoelectric element on one end and the expanded wire 101 on the other end. This approach would facilitate robotic placement of the cylinders and elements in the holes in an electronic assembly operation.

Yet another embodiment is to weave or assemble the string into a mold instead of the panel of FIGs. 3a-3d, then injection-mold the panel material into the mold. Upon removal of the mold, a similar configuration to FIGs. 3a-3d is obtained.

In the embodiment of FIGs. 3a-3d, the thermoelectric elements or tubes are spaced apart over a larger area vs. prior art modules, but the hot and cold sides are also separated by a length much longer than the elements. Since heat backflow conduction is proportional to area/length, scaling both simultaneously maintains a similar overall heat backflow as prior art thermoelectric modules. Since many desirable insulating panels like Styrofoam™, cloth, etc. have thermal conductivities comparable to air, the conduction ability of the invention's panel is comparable to that of the air cavity in prior art modules. In addition, the presence of the opaque panel blocks heat backflow from radiation almost entirely.

Once woven or placed, the exterior metal 101 in FIGs. 3a-3d is expanded, if necessary, on the hot and cold sides of the panel in order to maximize the exposure of the metal to air, which in turn maximizes its heat sinking or absorbing capability in either a natural or forced-air convection environment.

A key element of this invention over the prior art is re-optimizing the heat sinks for natural convection vs. the forced-air convection. With prior art forced-air convection systems, usually based on a fan, the forced air is moving in one direction only. Hence, the optimal heat sink is a metal plate for spreading the heat and linear metal "fins" for distributing the heat along the direction of the forced air. So, in prior- art forced air systems, the optimal heat sink maximizes the area touching air along the airflow, as represented by the parallel fins commonly used.

For a natural convection environment, the air flow velocity is much less than with a fan, but the air has the ability to move in all directions. Hence, the optimal heat sink for a natural convection environment is one that maximizes the area touching air in any direction.

In this preferred embodiment, re-optimizing the heat sink for natural convection brings about the following advantages: (1) better uniformity of the absorption of heat on the cold side and of the dissipation of heat on the hot side, (2) silent operation by eliminating the need for a fan, (3) much less total metal required, (4) more reliable because fans are prone to failure, (5) more efficient because the temperature change across the heat sink can be recovered to provide better additional cooling.

A typical prior-art thermoelectric module deployment has a heat sink with fins that are typically 2 mm thick. Because two surfaces of the fin are exposed to air, the total cross section perimeter of exposure is 4 mm for each thermoelectric element. In the preferred embodiment of this invention, the aggregate diameter d of the compacted wire is 1 mm. However, when the strands are spaced apart on the hot or cold side as 1/2 shown in FIGs. 3a-3d, the total cross section perimeter exposed to air is now N7i(d/N) where N is the number of strands and d is the aggregate diameter. As stranded wire is easily available with 100-400 strands, then total cross section exposed to air for the invention is 31.4- 62.8 mm, more than seven times the exposed cross section for prior art devices. Because of this larger cross section of exposure, the heat dissipation and absorption capacity of the invention can be, depending on geometric parameters, sufficient to eliminate the need for a fan as well as a rigid heat sink and rely instead only on natural convection. In addition, the larger amount of area touching air by the use of strands reduces the total amount of metal required for heat dissipation, facilitating lightweight, soft, and wearable panels.

Furthermore, the number of stranded wires in FIGs. 3a-3d may be increased almost arbitrarily while the diameter of each strand is proportionately decreased. As discussed above, more strands leads to increased heat absorption and dissipation by a factor N with natural convection. Thinner strands also allows for the heat sink of the invention to be soft, lightweight, and flexible in contrast to rigid, hard, and heavy heat sinks of the prior art. Wire braid of tinned copper with 72-400 strands is typically used in the electronics industry, and such braid is designed to be expandable in order to serve as shielding of cables of varying diameter. Each strand in these braids is AWG 36 or about -100 microns in diameter. Another type of braid, wick-braided copper, is used to remove solder and its strands are even thinner, making possible a very soft device for dissipating heat and carrying electrical current in a thermoelectric panel when the strands are spread apart. Copper mesh is also readily available with even thinner strands of 44 AWG and spread out in 140 strands per inch when fully expanded.

Without limitation, the panel 301 in FIGs. 3a-3d may be Styrofoam™, natural cloth, synthetic cloth, natural sponge, synthetic sponge, polyurethane, fiberglass, foam glass, building insulation material, wood, paper, cotton, batting, pipe-wrapping insulation, ceiling tile material, memory foam, cushion material, or any other insulating material.

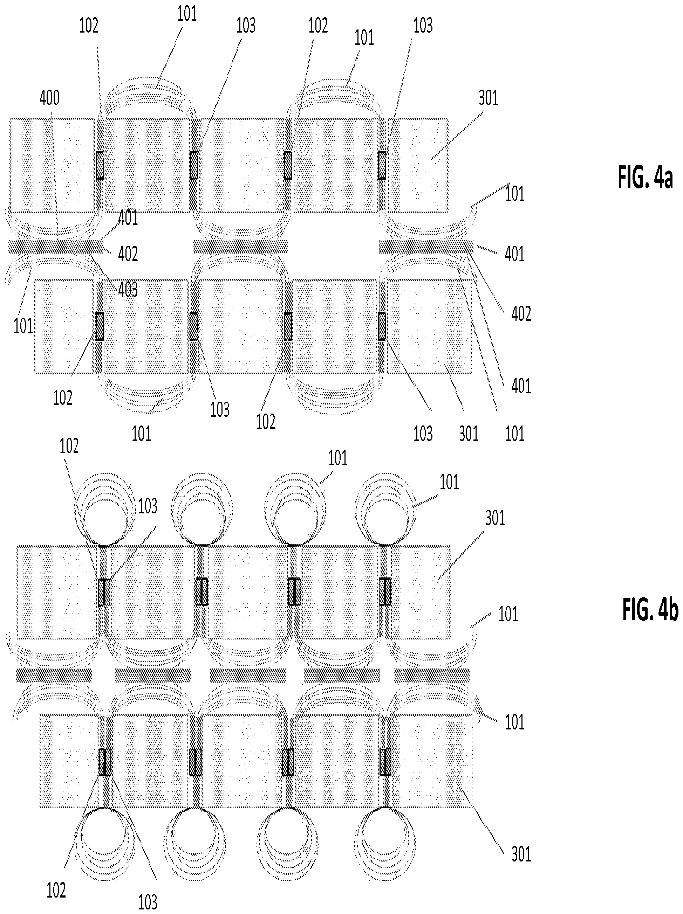

In some cases, it is desirable to have multi-stage thermoelectric cooling and heating. Higher temperature deltas are achievable. Prior art modules often are stacked together in a cascade configuration with 2 to 4 stages typically to achieve the very low temperatures needed for sensitive imaging cameras. The same multi-staging is possible with this invention and provides similar benefits, as illustrated in FIGs. 4a-4b. Here, two panels 301 are connected thermally in between by thermal connectors 400 that have high thermal conduction and electrical isolation. The thermal connectors may contain copper solder pads 401 and an electrically insulating layer like polyimide 402. In this configuration, the polyimide layer 402 is so thin that its thermal conduction is high. Without limitation, the electrical insulator could be FR-4, Kapton, Teflon, an insulated metal substrate circuit board, aluminum oxide or any other readily available material. The multi-stage configuration may be applied to the alternating weave as shown in FIG. 4a or to the single-sided weave as shown in FIG. 4b. The thermoelectric elements are shown as pellets 102 and 103 but could also be thermo-tunneling tubes 203 and 204 shown in FIGs. 2a-2b and 3a-3d.

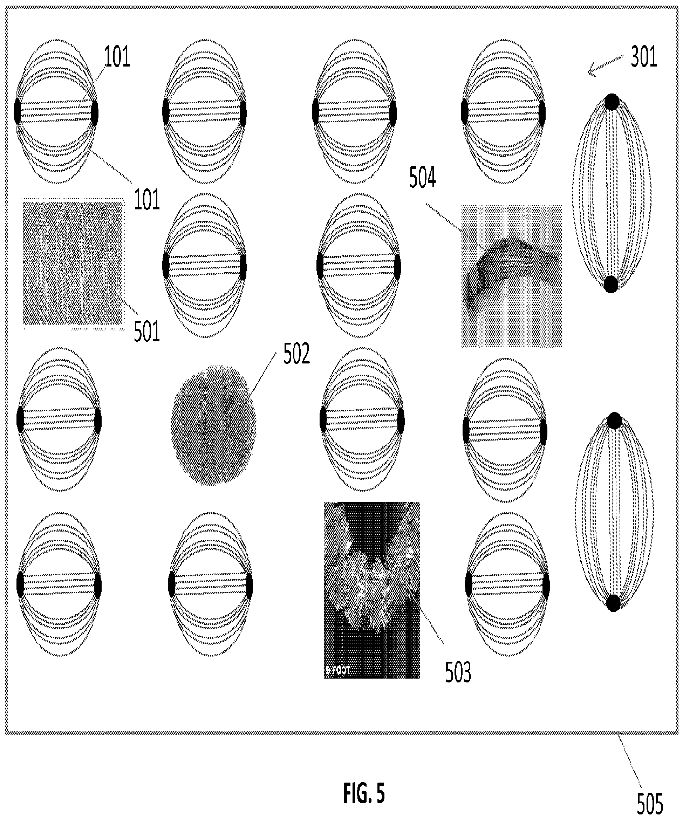

FIG. 5 shows several different types of expandable metal conductors that may replace the braid 101 in FIGs. 1a and 3a and 3d, and 4a and 4b. Copper mesh is available in an oriented form 501 or un-oriented form 502 and either provides strands with high contact area to air. Metal tinsel 503 has a thick central wire which is convenient for moving electricity from one thermoelectric element to the other plus many branches of thin copper strands which are convenient for dissipating or absorbing heat to or from the air. Flat braid 504 is also available with or without solder joints on either end. A panel made with one or a combination of these expanded metals 505 becomes a fully functional thermoelectric panel.

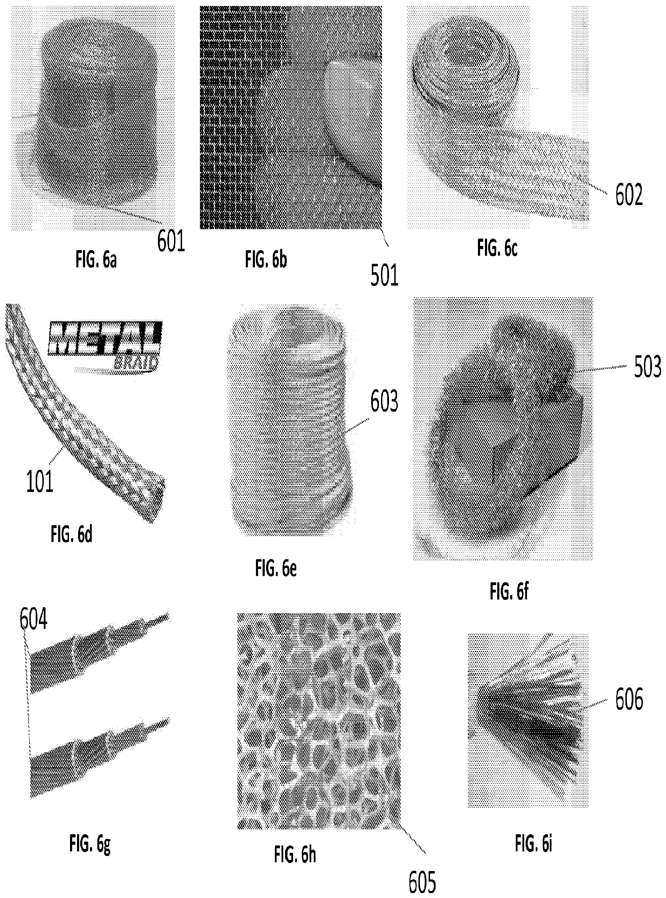

FIGs. 6a-6i show even more possibilities for expanded or expandable metals, including another type of un-oriented copper mesh 601 , copper strands weaved like rope 603, coaxially grouped strands 604, copper foam 605, or loose copper strands 606. For the metal screen or mesh, the metal may be compacted by rolling tightly or folding tightly in an accordion shape near the thermoelectric elements, and loosening the roll or the folds away from the thermoelectric elements.

The thermoelectric panels described can also be deployed for generating electricity from heat. When heat is applied to one side, a Seebeck voltage is generated that can be used for electrical power. The heat source can be a selective surface receiving sunlight, a road or highway surface, geothermal heat, engine heat, smokestack heat, body heat, waste heat, and many other possibilities.

### Example 1 : A Thermoelectric Cooler using Invention

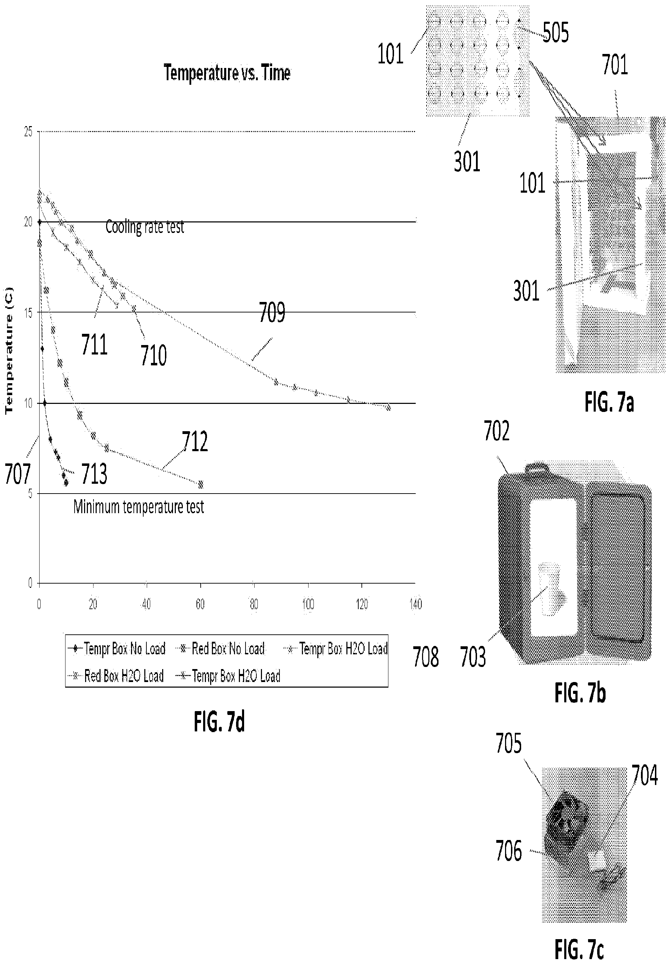

FIGs. 7a- 7c illustrate a thermoelectric cooler 701 using the invention. Four thermoelectric panels 505 were built using a string as shown in FIG. I a with braid 101 lengths 7 and 1 1 cm for the cold and hot sides, respectively. The panels were 1-inch (2.54 cm) thick Styrofoam™ 301 with 3 mm diameter holes and a pellet spacing of 3 cm. A total of 256 pellets were inserted into the four populated panels. The four thermoelectric panels were combined with two plain Styrofoam™ panels to construct a small cooler. The cooler 701 in FIGs. 7a-7c did not contain a heat sink or a fan and was powered with 20 watts of electricity.

The cooler of FIGs. 7a-7c was compared with a prior art commercial cooler 702 that contains a prior art thermoelectric module 704 also with 256 pellets, a prior art heat sink 706, and a prior art fan 705. This commercial cooler was powered as designed with 40 watts of electricity.

FIG. 7d shows the data taken during an experiment to compare the embodiment cooler with the prior art commercial cooler. The two key measures of performance for such a cooler are (1) the rate of cool-down for a roomtemperature cup of water 703 and (2) the minimum temperature reached by the air inside each cooler. The graph 707 in FIG. 7d plots the temperature on the Y-axis and the elapsed time in minutes on the X-axis.

The experiment revealed that the cooling-down rate for the cup of water, indicated by the slope of the line 709 and 71 1 for the embodiment, was comparable to the cooling-down rate of the prior art commercial cooler, indicated by the slope of 710. In addition, the minimum temperature of the air inside the box reached 5.5 degrees C for both the invention cooler as indicated by line 713 and for the prior art cooler 712.

The data in FIG. 7d indicates that the embodiment performs as well as the prior art commercial cooler in cooling. However, it only required 20 watts of power vs. 40 watts for the prior art commercial cooler. Hence, the embodiment achieved the comparable performance with significantly greater efficiency. The greater efficiency is due to the following: (1) not requiring the electrical power for a fan, (2) recovery of much of the temperature drop across the heat sink, and (3) better distribution of the cooling over the walls of the container.

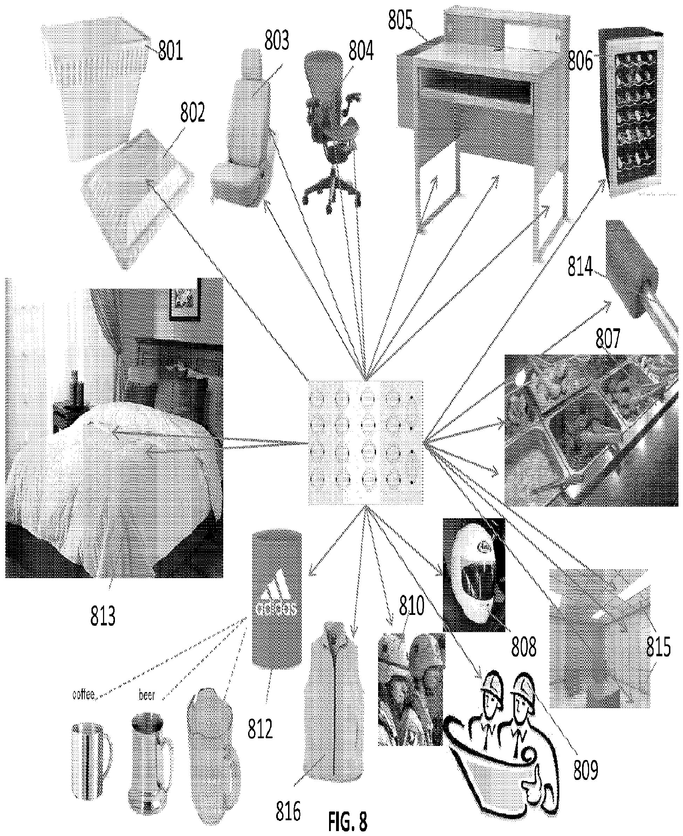

The thermoelectric panels of the embodiment illustrated in FIGs. 3a-3d and 4a and 4b are generalized insulating panels with the ability to cool or heat one side relative to the other. These generalized panels may be manufactured using a similar process and with similar machines and then deployed in a plurality of applications. Without exception, some of these applications are illustrated in FIG. 8.

In order to save overall energy or achieve greater individual comfort in cooling or heating the human body, one advantageous technique is to allow for local heating or cooling relative the environment. For example, the thermoelectric panel may be placed around the cavity under a desk 805 as illustrated in FIG. 8 to provide local comfort for an office worker with significant energy savings. Or, the panel could be placed in an office chair 804 in the seat bottom or the seat back or both. In a vehicle, the panels may be placed in the seat bottom or seat back of a car seat 803. For sleeping, these panels may be placed in an electric blanket 813 combined with a thermostatic controller to maintain a desired under-blanket sleep temperature. The control electronics for the blanket can automatically switch the electrical current in the proper direction when cooling is needed to achieve the set temperature or when heating is needed. Without limitation, such thermostatic control can be applied to any of the applications of the invention including all of those illustrated in FIG. 8.

For individuals that must wear helmets, the body heat confined inside the helmet can be uncomfortable. Or, the helmet may not provide sufficient warmth when worn in cold environments that require head protection. The thermoelectric panel may be molded into the proper shape to add cooling and heating capability to helmets of all types, including motorcycle or bicycle 808, military 810, or hard hats 809 for construction sites.

Similarly, the present panel may be shaped and used to make clothing like vests 816 or, without limitation, other types of clothing such as coats, pants, pant legs, and shirts.

The thermoelectric panel also can be used to cool food and drinks or other objects. These panels can be deployed as the wall, door, back, or top of a wine chiller 806 or a camping cooler 801 and 802. Because the panel and string can be flexible 812 in FIG. 8, it can be wrapped around shaped objects like water pitchers, beer or other mug or bottles, coffee drinks, milk or cream bottles or cartons, etc.

The thermoelectric panel also may be deployed to heat or cool buffet trays 807 shown in FIG. 8 for self-serve restaurants, cafeterias, or catering services. The prior art uses ice to cool the trays and boiling water to heat them. The supply of ice and hot water must be maintained and the reservoir under the trays must be replenished periodically. The present panel provides benefits over the prior art by heating or cooling the trays electrically and not requiring cold and hot supplies.

The thermoelectric panel also may be deployed in residences and buildings. A portion of a wall or window or floor 815 may be replaced by the panel and provide heating or cooling for room. The ceiling tiles 815 in buildings also may be replaced by the panels to provide heating and cooling for the space underneath the ceiling. The present panel also may be employed in combination with central compressor-based air conditioning systems to eliminate the need for forced air that can carry germs and smells from one room to another. In this case, the panels would be mounted along plenums with the hot side facing into the plenum. The cool air from the compressor-based HVAC system would carry the heat away from the hot side while the cold side of the panel removes heat from the room. In this case, the room is cooled without forced air.

In another embodiment, the invention provides renewable electrical power from the sun's radiation in well-suited climates. A second purpose is to continue providing energy when the sun is not shining and all night long. A third purpose is to improve the land utilization as measured in Kwatts/acre to many times higher than a wind turbine farm. A fourth purpose is to provide peak power capacity at a time of day that better matches the typical peak demand time for electricity. A fifth purpose of this invention is to use inert and non-toxic materials to store the energy of the sun in the form of heat. A sixth purpose is to provide these capabilities at a cost per watt that is a fraction of the cost (including fuel costs) of a traditional power plant and an even smaller fraction of the cost per watt of a PV or wind turbine plant (including battery storage costs). As discussed below, the embodiment demonstrates better performance over prior art implementations that do not have energy storage such as US 3,088,989, by additionally distributing the thermoelectric elements to match the heat distribution from un- concentrated sunlight and remove the need for metal heat spreaders.

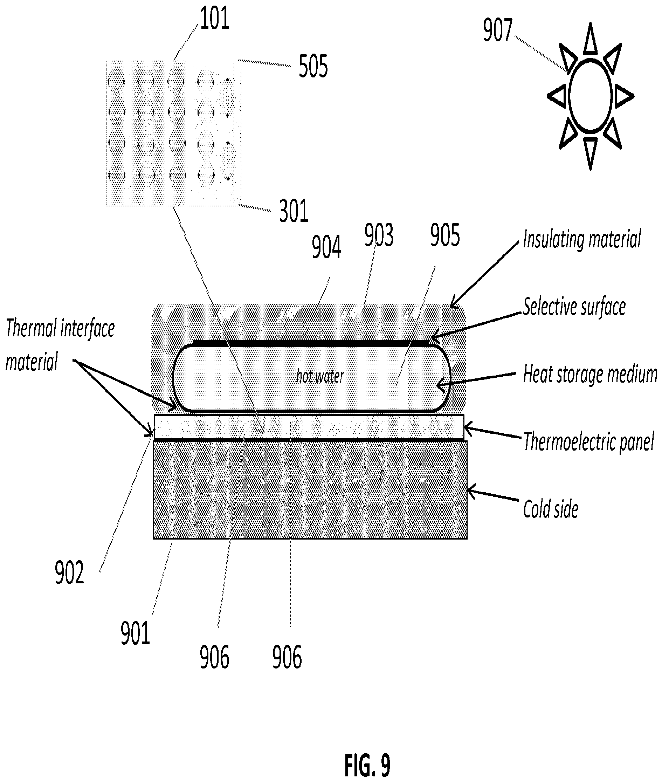

An embodiment of the invention is illustrated in FIG. 9. An insulating material 903 that is largely transparent to the sun's radiation surrounds heat storage medium 905. The insulating material 903 also prevents the heat from escaping when the sun 907 is not shining. The insulating material may be, without limitation, bubble wrap, glass or Plexiglas sealing in air or air pockets, or any of the materials used for solar covers for swimming pools. A selective surface layer or coating 904 of the heat storage medium is designed to absorb radiation from the sun and prevent radiative re-emission of absorbed heat. This selective surface layer or coating 904 may be constructed, without limitation, from, e.g. an oxide of copper, aluminum, or iron, from carbon, steel or a combination or alloy of these, black paint, or similar materials used in solar ovens, solar camping showers, or solar rooftop water heaters. The heat storage medium 905 contains a large volume of a material with a high heat capacity . This material could be water, which has a volumetric heat capacity of 4.2 joules/cm3/°C or could be scrap iron which has a heat capacity slightly less than water. The selective surface 904 and the heat storage medium 905 are in good thermal contact. This contact possibly employs a thermal interface material 906 there between that has high thermal conductivity, the ability to mate the surfaces, and the ability to spread the heat. The heat storage medium 905 is thermally connected to the hot side of a distributed thermoelectric panel 902, again possibly employing a thermal interface material 906. The distributed thermoelectric panel 902 is an insulating panel with thermoelectric elements inside, as described in FIGs. 2a and 2b and FIGs. 3a-3d. The cold side of the thermoelectric panel 902 is thermally connected to ground 901 or floating on a body of water such as an ocean, lake, or pool.

Without limitation, the power generator illustrated in FIG. 9 could generate power only when the sun 907 is shining, eliminating the need for storage medium 905. In this case the selective surface 904 would be adjacent to the thermoelectric panel 902, possibly with a thermal interface material 906 there between,

Again without limitation, the power generator of FIG. 9 could employ a heat source other than sunlight. The water in the storage medium 905 could flow from an active geothermal source, or be heated waste water from a power plant or factory. If the thermoelectric panel 902 were built in the flexible configuration described earlier, then it could be wrapped around pipes carrying hot water or hot gases and generate electricity as illustrated in FIG. 8, item 814.

### Example 2: Solar Power Storage and Electricity Generation

An example power generator in accordance with FIG. 9 will now be described that is competitive with other power generators such as wind turbines and photovoltaic panels. The heat storage medium 905 is 2m x 2m x 0.3m and is assumed to reach a peak temperature of 100°C. This temperature does not exceed the boiling point of water, and is a temperature easily reached by insulated solar ovens used to cook food. The cold side 901 temperature is assumed to be room temperature or 20°C. The delta temperature ΔT across the thermoelectric panel 902 is then 80°C and the average temperature is 60°C. The heat storage medium at a temperature elevated by 80°C relative to ambient stores 4.0 E+8 joules or 1 12 Kwatt-hours if the heat capacity of water at 4.2 joules/cm3°C is assumed.

The insulating material 903 dimensions are 2m x 2m x 0.05m, and so the thermal loss through the thickness of the insulator at the ΔT of 80°C is 147 watts if a typical thermal conductivity of air-pocket insulators of 0.023 watts/m°C is assumed.

Thermoelectric elements are readily available with an electrical resistance r of 0.005 ohm, thermal conductance K of 0.009 watts/°C, and Seebeck coefficient S of 300µv/ °C. These values indicate a thermoelectric performance ZT =S2T/rK at the average temperature of 60°C (333K) of 0.60, which is well within the performance claimed by most manufacturers. The distributed thermoelectric panel 902 is 2m x 2m x 0.05m, and it contains 1333 thermoelectric elements. The elements are spaced apart by 5.5 cm in each lateral direction. The total thermal loss through the elements is 960 watts (1333ΔTK). The total voltage V generated by the elements connected in series is I 338ΔT or 32 volts. The total resistance of the elements, all connected in series, is R = 1333r = 6.7 ohm. Assuming a matched load of 6.7 ohm, then the current flow I is V/2R or 2.4 amps. Hence, a total of 38.4 watts (0.5VI) of power is available to the load by this example embodiment.

The sun's 907 radiation is known to be about 1000 watts/m2, which indicates that 4000 watts reaches the selective surface 904. After subtracting the loss through the thermoelectric elements and through the insulating material, 2893 watts (4000-960- 147) is absorbed as heat in the heat storage medium 905. Because 4000 watts are entering the medium for 8 hours of the day and 1145.4 watts (960+147+38.4) are leaving the medium for 24 hours of the day, more energy (net 4.52 Kwatt hours per day) is entering per day than is leaving, allowing for this embodiment to reach and maintain a maximum temperature. The heat builds up in the heat storage medium until it reaches its heat capacity of 112 Kwatt hours. The time required to reach the maximum temperature is about 25 days (112 Kwatt hours/4.52 Kwatt hours per day).

While this embodiment is less than 1% efficient on an instantaneous basis (38.4 watts generates/4000 watts available from the sun), which is a conservative expectation for a thermoelectric generator at these temperatures, making use of the heat storage allows the thermoelectric device to be about 3% efficient on a daily average basis.

A feature and advantage of this embodiment is that it reaches its maximum temperature in the mid-afternoon hours as heat builds up in the heat storage medium 905. Hence, the time of maximum power output of this embodiment better matches the time of peak demand for electricity. Photovoltaic panels have their maximum output at noon, which is two hours earlier than the peak demand. The daily maximum output of wind turbines is unpredictable.

With this embodiment, 38.4 watts of electrical power generated in a 2m x 2m area corresponds to 38 Kwatts per acre, which compares very favorably to wind turbines which average about 4 Kwatts per acre.

Another feature and advantage of the present invention is that the storage medium, water, of this embodiment, is essentially free as the water does not even need to be fresh water. Storing energy as heat is much less costly than storing energy as electricity, and it may be stored without the toxic chemicals found in batteries.

### Example 3: A Distributed Thermoelectric Mattress

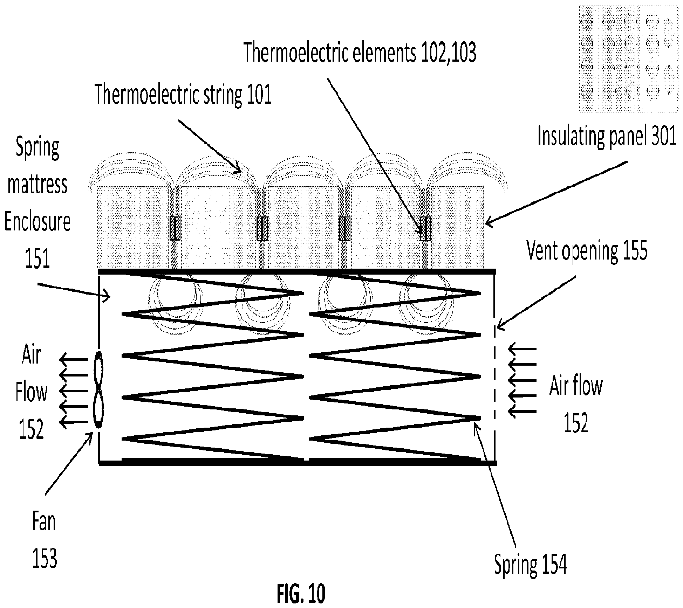

FIG. 10 illustrates how the thermoelectric panel of PIG. 3c can be used to heat or cool the surface of a mattress with springs. The braided or stranded wire of the thermoelectric string 101 extends into the cavity of the mattress enclosure 151 . A fan 153 is used to move heat away from or toward these wires, depending on whether mattress surface is being heated or cooled. Because the heat is highly distributed by the invention, the fan can have much lower revolutions per second to keep down noise and power consumption. In some cases, a fan may not be needed at all if the cavity of the mattress is well ventilated by other means. The air flow 152 generated by fan 153 sees little resistance from the presence of the springs 154. A vent 155 allows the air from fan 153 to escape to the environment.

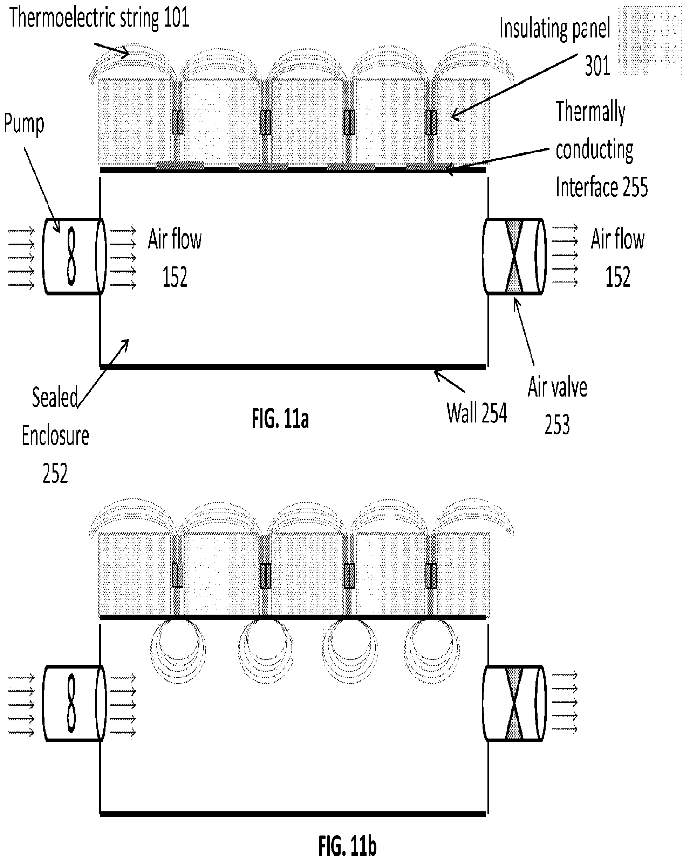

FIGs. 11a and 11b illustrate a similar concept for an air mattress. The air pressure in the mattress might be controllable to provide varying amounts of firmness or might be fixed. A pump 251 is run continuously to remove or insert heat again depending on whether the mattress surface is being cooled or heated. In FIG. 11a, a thermal connection is made through the wall 254 of the air mattress by a thermally conducting interface 255. In FIG. 11b, the braided or stranded wire extends through holes in the wall 254 of the air mattress in order to make contact with the convective airflow 152 from pump 251.

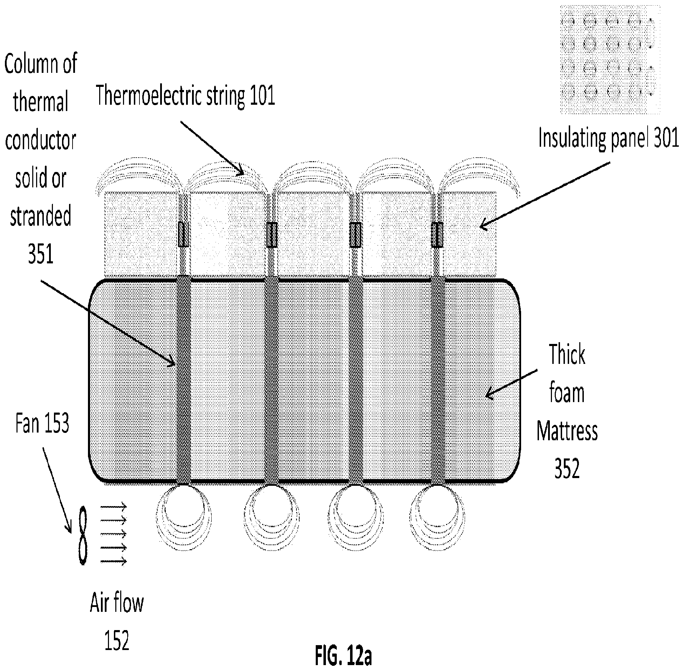

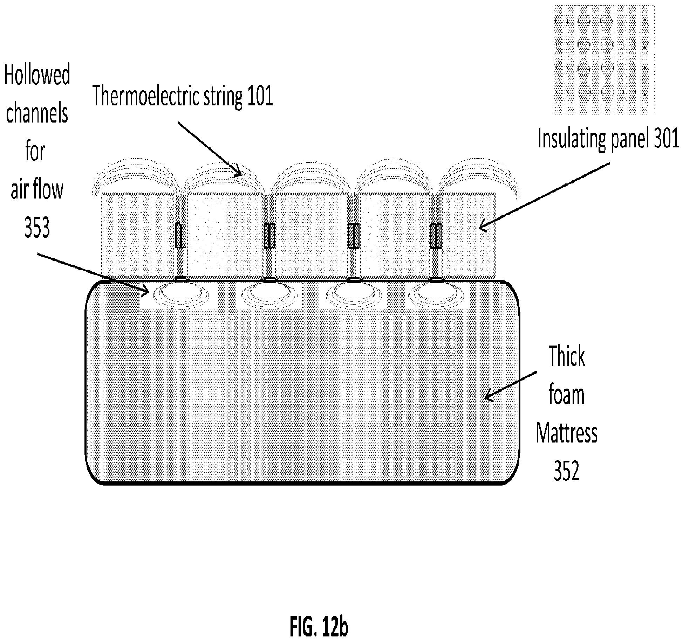

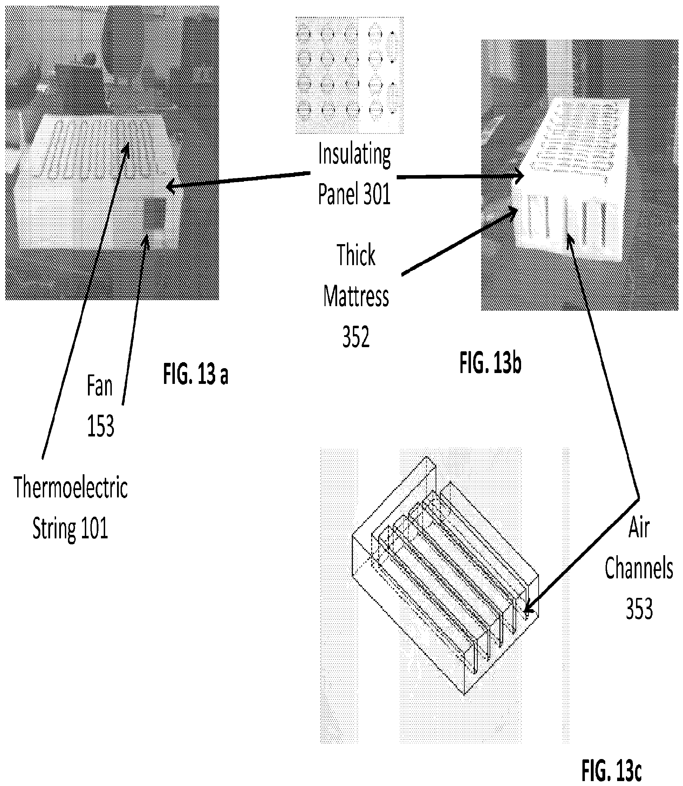

FIGs.12a and 12b illustrates a similar concept for a thick foam mattress 352. In FIG. 12a, thermally conductive columns 351 are used to thermally connect down through the thickness of the mattress to underneath the bed where convective air flow- exists. A fan 153, if necessary, can supplement the natural convection. FIG. 12b employs hollow channels 353 in the foam mattress 352 to provide a convective path for air flow. These hollow channels may be lined with soft or hard pipes to restore the rigidity lost by the hollowed areas. A fan, not shown, if needed, is used to move convective air across the stranded or braided wire. Again, the fan can be very low speed because the heat is already highly distributed by the invention. Without limitation, the top portion of the mattress arrangement in FIG. 12b could be a topper for any mattress, thereby providing heating or cooling to that mattress without requiring modification to the mattress. Similarly, smaller sections of the arrangement in FIG. 12b could be deployed as seat cushions or seat back cushions to bring heating or cooling to any chair without requiring modifications to the chair. FIGSs. 13a- 13c illustrate a similar concept for a thick foam mattress 352 in which air channels 353 are cut out of the foam. FIG. 13c shows a drawing of a thick foam mattress with the air channels 353 cut out of the foam 352. The channels 353 running the length of the mattress are all connected to a lateral channel that provides air to all of them. The thickness and depth of the channels can be designed to equalize the airflow in each channel appropriately. FIG. 13a and 13b show two pictures taken at different angles of a prototype mattress built in accordance with drawing 13c. A thermoelectric panel 301 is placed on top of the mattress with the hollowed channels with the braided or stranded wire exposed to the convective airflow from the fan 153 to the ends of the channels 353.

### Example 4: A Distributed Thermoelectric Blanket

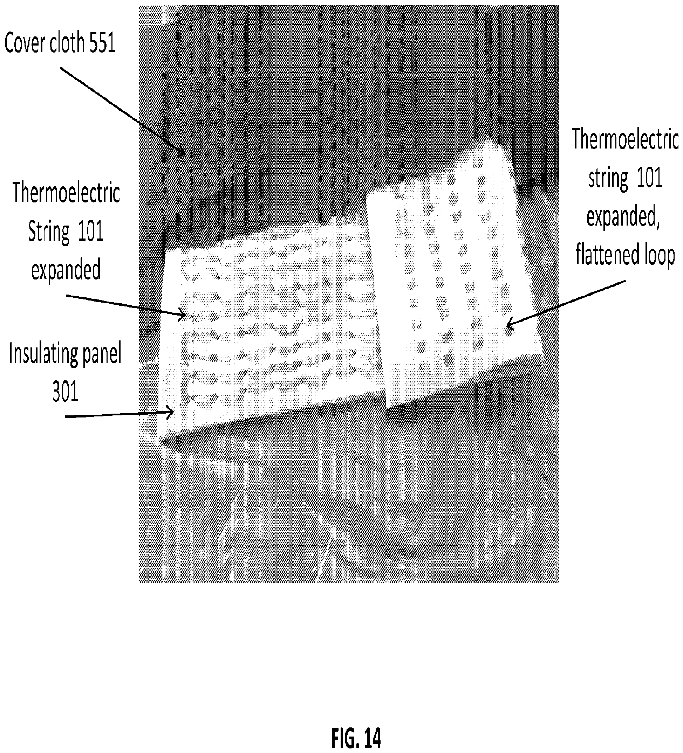

FIG. 14 shows a picture of the invention thermoelectric panel deployed as an electric blanket that heats and cools. The panel insulating material 301 is soft and light memory foam, but without limitation could be batting or other types of foam. The braided wires of the thermoelectric string 101 are shown on each side. A cover cloth 551 is used to cover the appearance and the feeling of the braided wire. This cover cloth needs to transmit heat effectively and hence may be comprised of a material with low thermal conductivity but very porous such as cotton, linen, or polyester, or be comprised of a material with high thermal conductivity but not very porous such as carbon impregnated films, or be comprised of a phase change material that moves heat through the changing of a phase from solid to liquid or from liquid to gas. Phase change fabrics such as Outlast are readily available for this purpose. Note how the thermoelectric effect in contact with one side of the phase change material allows it to be continually effective with continuous phase change cycles taking place over time vs. the single cycle of the material without the thermoelectric effect. Without limitation, this phase change material may be combined with any deployment of the distributed thermoelectric panel including all those illustrated in FIG. 8.

### Example 5: A Distributed Thermoelectric Chair

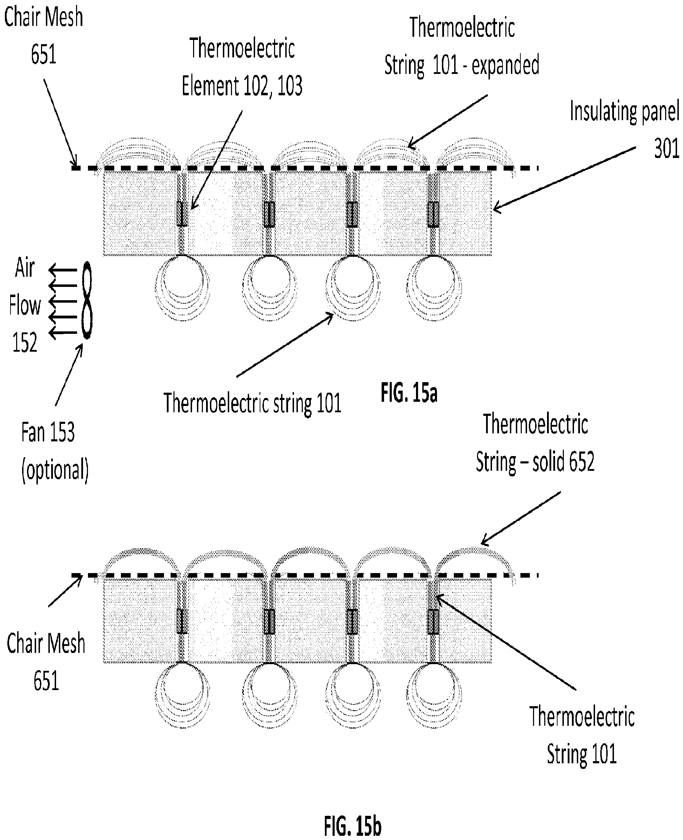

FIGs. 15a and 15b shows how the invention thermoelectric panel may be integrated with a mesh-style office chair. In these types of chairs, the mesh 651 supports the load and the distribution of pressure for the seated person. The intention of FIGs. 15a and 15b is to illustrate one embodiment of the thermoelectric panel in which heating or cooling is added to the chair's function without changing the structural or comfort properties of the original chair. Another objective is to have the braided or stranded wire of the expanded thermoelectric string 101 as close to the skin or clothing as possible in order to achieve a good thermal connection. For this reason, the wires are brought through the mesh 651 in their original compacted form, and then the wires are expanded on the side of the mesh 651 that contacts the skin or clothing. Without limitation, the wires could provide heating or cooling without being brought through the mesh, or the mesh could be made from a high thermal conductivity material, or the mesh could be a phase change material. If the wires of FIGs. 15a and 15b are in good contact with the skin, then heat may be conducted with a solid wire 652 as shown in FIG. 15b instead of stranded or braided wire shown in FIG 15a. Also, if the desire is to insulate the wire to prevent shorting, then magnet wire or Litz wire can be used in place of un-insulated wire.

FIGs. 16a-16c show pictures of a mesh style office chair that was built according to FIGs. 15a and 15b. FIG. 16a shows a chair in which the braided wire panel insulating material 301 (FIG. 16d) of the thermoelectric string 101 is behind the mesh of the back of the chair. Without limitation, the same technique could be used to heat and cool the seat. FIG. 16b shows another chair in which the braided wire of the thermoelectric string 101 is brought through the mesh 651 to be in better contact with the skin. FIG. 16c shows the braid of the thermoelectric string 101 on the back side of the chair fully expanded with the braids exposed to open air for maximum effect of natural convection.

### Example 6: Thermoelectric Panel with Electronic Control

FIG 17 shows a schematic of a control circuit that may be used to power and control the panel for all of the aforementioned applications of this invention. Power supplies 851 with variable voltage output are readily available in the computer industry such that one supply can be configured to power multiple laptop computers. These universal power supplies are available from IGO and other manufacturers, and they have an output with three wires: two wires supply the power and a third wire 852 senses a voltage level that determines the voltage output. When used in laptop computers, the desired control voltage is determined by a "tip" that sets the control voltage. In the implementation of FIG. 17, such a universal power supply is used to allow the user of the thermoelectric panel 301 to set a desired amount of heating or cooling.

The DPDT switch 853 in FIG. 17 sets the polarity of the current flow in the panel, hence allowing the user to select heating or cooling. The middle position of the DPDT switch 853 provides no connection and hence is used for the off position. The ganged potentiometers 857 determine the control voltage sent back to the universal power supply 851 and hence allow the user to set how much heating or cooling is provided by the panel. The presence of thermistor 855 raises the control voltage and hence increases cooling when the ambient temperature rises, and equivalently lowers this voltage to decrease cooling when the ambient temperature drops. The presence of thermistor 856 increases the control voltage and hence increases heating when the ambient temperature drops, and equivalently decreases the control voltage and decreases heating when the ambient temperature rises. Trimming potentiometers 856 set the minimum cooling, maximum cooling, minimum heating, and maximum heating that the panel 301 is allowed to generate. The intent is for these trimming potentiometers to be set at the factory to safe or otherwise desired levels. Diodes 858 ensure that only one of outputs of the ganged potentiometers 857 is able to set the control voltage 852.

## Claims

1. A string of thermoelectric elements (102, 103) **characterised in that** the thermoelectric elements (102,103) are connected to one another by stranded wire conductors (101), wherein each of the stranded wire conductors (101) is compacted in cross section near where the stranded wire conductors (101) connect to the elements (102, 103) and is expanded in cross section away from where the stranded wire conductors (101) connect to the elements (102, 103).

2. The string of claim 1, **characterized by** one or more of the following features:
(a) wherein each of the stranded wire conductors (101) is formed as metal strands, braid, mesh (501), screen, tinsel (503), or foam (605);
(b) wherein each of the stranded wire conductors (101) is formed of metal formed as strands which are loose, roped, or grouped axially;
(c) wherein each of the stranded wire conductors (101) is formed of metal formed as strands which are squeezed together for compaction near the element and spread apart for expansion away from the elements (102, 103);
(d) wherein each of the stranded wire conductors (101) is formed of metal formed as a braid which is woven in a round, oval, or a flat tubular shape, and wherein the tubular shape braid preferably has a first portion located adjacent to the elements (102, 103) and a second portion having a diameter or width that is greater than the diameter or width of the first portion and located farther away from the elements (102, 103) than the first portion;
(e) wherein each of the stranded wire conductors (101) is formed of a metal mesh or metal screen which is folded in a tight accordion shape adjacent the elements (102, 103) and in a loose accordion shape away from the elements (102,103);
(f) wherein each of the stranded wire conductors (101) is formed of a metal mesh or screen having a first portion located adjacent to the element (102, 103) and a second portion that is more loosely rolled than the first portion and is located farther away from the element than the tightly rolled portion;
(g) wherein the thermoelectric elements (102, 103) are comprised of a material selected from the group consisting of a semiconductor material, a doped semiconductor material, lead, bismuth, antimony, selenium, tellurium, a thermo-tunneling vacuum tube and a combination thereof;
(h) wherein the thermoelectric elements (102, 103) comprise alternating N and P type elements (102, 103); and
(i) optionally further including strain relief (106) for mounting and protecting the thermoelectric elements (102, 103), wherein (i) the strain relief (106) preferably is cut from or assembled from circuit board substrate material, and wherein the circuit board substrate material preferably is selected from the group consisting of a polyimide, Kapton, polyester, nylon, FR-4, epoxy, glue, and fiberglass or fiberglass cloth adjacent to or surrounding the thermoelectric elements (102, 103) and a portion of the compacted conductor; or (ii) wherein the strain relief (106) comprises copper or other metallic pads for solder-attaching the compacted metal to the strain relief (106) and to the thermoelectric element, and/or
(j) optionally with a variable power supply that is controlled by the user, preferably a universal power supply with a control voltage set by a potentiometer adjustable by the user, and optionally including a polarity switch for selecting heating or cooling, and/or a thermistor that adjusts the control voltage in response to changes in ambient temperature.

3. A thermoelectric panel **characterised in that** it comprises the string of claim 1 or 2, wherein the thermoelectric elements (102, 103) are woven in and out of holes (302) in an insulating panel (301) wherein portions (303) of the metal within the holes (302) in the panel (301) are mostly compacted and portions outside the holes (302) in the panel (301) are mostly expanded, or pairs of thermoelectric elements (102, 103) having metal therebetween are pushed through a hole from one side of an insulating panel (301) exposing a loop of expanded or expandable metal on the other side and retaining the elements (102, 103) within the panel (301), wherein the panel (301) preferably is made of a material selected from the group consisting of Styrofoam™, natural cloth, synthetic cloth, natural sponge, synthetic sponge, polyurethane, fiberglass, foam glass, building insulation material, wood, paper, cotton, batting, pipe-wrapping insulation, ceiling tile material, memory foam, and a cushion material.

4. A thermoelectric device **characterised in that** it comprises a plurality of thermoelectric panels according to claim 3 that are stacked together in ascending or descending thermal order to achieve larger temperature differences.

5. The thermoelectric device of claim 4, **characterized by** one or more of the following features:
(a) wherein the plurality is a whole number equal to 2, 3, or 4;
(b) wherein a plurality of string and panel (301) assemblies are connected together and electrically isolated on a thermally conducting board or group of boards, preferably a circuit board or group of circuit boards (401);
(c) wherein a plurality of string and panel (301) assemblies are connected together and electrically isolated on thermally conducting boards by an electrical isolation material selected from the group consisting of FR4, Kapton and a polyimide;
(d) wherein a plurality of string and panel (301) assemblies are connected together and electrically isolated on thermally conducting boards (401) by an electrical isolation material which is thin or contains a metal substrate with thin isolation layers to permit high thermal conduction, and wherein the thin electrical isolation material preferably is Kapton, a polyimide or an oxide of a metal substrate, and has a thickness of 10 to 40 microns;
(e) containing copper or other metallic pads to facilitate soldering of the expanded metal outside the stacked panel (301)s on either side of the board or boards.

6. A method for forming a thermoelectric panel according to claim 3, **characterised in that** it comprises the steps of:
(a) assembling a plurality of thermoelectric elements (102, 103) as in claim 1 on a substrate board patterned with pads of strain relief (106), and cutting the plurality of thermoelectric elements (102, 103) with strain relief (106) assemblies from the substrate board, or
(b) weaving a plurality of thermoelectric elements (102, 103) as in claim 1 in and out of holes (302) in an insulating panel (301), wherein portions of the stranded wire conductors (101) within the holes (302) in the panel (301) are mostly compacted and portions outside the holes (302) in the panel (301) are mostly expanded, which comprises weaving strings of thermoelectric elements (102, 103) in a mold, injecting a settable panel material into the mold, allowing the panel material to set, and removing the mold.

7. The thermoelectric panel of claim 3, wherein the thermoelectric panel is configured to generate electricity when heat is applied to one side of the thermoelectric panel, wherein the heat preferably comprises a heat source selected from the group consisting of sunlight, geothermal heat, waste heat, body heat, animal heat, exhaust heat, engine heat, turbine heat, and pipe heat.

8. An apparatus for storing energy from the sun and generating electricity from said stored energy, **characterised in that** said apparatus comprises a transparent insulator (903), a selective surface (904), a heat storage medium (905), a distributed thermoelectric panel (902) and a cold reservoir, wherein the distributed thermoelectric panel (902) includes said at least one string of thermoelectric elements (102, 103) as defined in claim 1.

9. The apparatus of claim 8, **characterized by** one or more of the following features:
(a) wherein the transparent insulator (903) is comprised of a largely clear solid and air, wherein the solid preferably is formed of a material selected from the group consisting of polyethylene, plexi-glass and glass, and/or wherein the solid optionally forms pockets surrounding the air or evacuated regions;
(b) wherein the selective surface (904) comprises a metal oxide, an anodized metal, black paint, or a combination thereof;
(c) wherein the selective surface (904) is selected from the group consisting of carbon, copper, aluminum, iron, steel and combination or alloy thereof;
(d) wherein the heat storage medium (905) comprises a material with high heat capacity, wherein the material preferably is selected from water, iron, asphalt, glass, sand, salt, copper and a combination thereof;
(e) wherein the heat storage medium (905) is enclosed in a container, wherein the container preferably is formed of vinyl;
(f) wherein the distributed thermoelectric panel (902) is thermally connected to the heat storage medium (905) on one side and thermally connected to the cold reservoir on the other side, wherein the distributed thermoelectric panel (902) preferably includes n- and p- type thermoelectric elements (102, 103) connected electrically, and/or wherein the thermoelectric elements (102, 103) are thermo-tunneling devices;
(g) wherein the distributed thermoelectric panel (902) includes a vacuum cavity;
(h) wherein the cold reservoir is selected from the group consisting of the ground, soil, sand, a natural body of water, and a man-made body water; and
(i) further comprising a thermal interface material on one or both sides of the distributed thermoelectric panel (902).

10. The use of a string of thermoelectric elements (102, 103) as defined in claim 1, **characterised by** the string being incorporated into a seat cushion, seat back, blanket or blanket section, pillow, under-desk panel, ceiling tile, building or residence wall or floor or window, refrigerator or wine chiller wall or door, beverage or pitcher insulator, electronic enclosure wall, piece of wearable clothing or uniform, a helmet or a hat or a hardhat lining or a pipe containing fluid.

11. Use of the thermoelectric panel as defined in claim 3 in a mattress, **characterised by** the panel being mounted on top of the mattress, wherein
(a) the mattress is a spring mattress, and a portion of each of the stranded wire conductors (101) is exposed in the cavity containing the springs and forced or natural convection of air is available in said cavity; or
(b) the mattress is an air mattress and the thermoelectric device is mounted on top of the air mattress, and includes a thermal connection of each of the stranded wire conductors (101) on one side of the device into the cavity containing the air and movement of the air is available in said cavity; or
(c) the mattress is a foam mattress and the thermoelectric device is mounted on top of the thick foam mattress in which a portion of each of the stranded wire conductors (101) extends into hollowed channels that provide natural or forced convection of air; and optionally
(d) wherein the mattress is used on top of another mattress or sofa or couch or seat bottom or seat back as an accessory; and/or
(e) wherein the holes (302) also contain pipes or tubes for additional support; and/or
(f) further including a fan or pump to provide forced convection of air.

12. Use of the thermoelectric panel as defined in claim 3, **characterised by** the panel being mounted on the seat bottom or seat back or both of a chair, sofa, ottoman, wheelchair, pillow, or couch, and wherein the thermoelectric panel preferably is mounted behind or underneath the existing support mesh in the back or bottom, and wherein a portion of each of the stranded wire conductors (101) of the thermoelectric panel preferably protrudes through the mesh to achieve better contact with the skin or clothing.

13. Use of a thermoelectric panel as defined in claim 3 in a mattress, furniture, blanket, pillow or clothing including but not limited to a hat or helmet, **characterised in that** a cover cloth is provided over each of the stranded wire conductors (101) on the side of the thermoelectric panel (301) that contacts the skin or clothing, wherein the cover cloth preferably is comprised of natural or synthetic fabric that is thin and porous and allows air flow, or is comprised of a fabric, film, or mesh that is designed to have high thermal conductivity, or is comprised of a material that changes its phase when in contact with human or animal skin thereby moving or removing heat.

## Patentansprüche

1. Kette aus thermoelektrischen Elementen (102, 103), **dadurch gekennzeichnet, dass** die thermoelektrischen Elemente (102, 103) über Drahtlitzenleiter (101) miteinander verbunden sind, wobei die Drahtlitzenleiter (101) jeweils nahe der Stelle, an der die Drahtlitzenleiter (101) mit den Elementen (102, 103) verbunden sind, im Querschnitt verdichtet sind, und entfernt von der Stelle, an der die Drahtlitzenleiter (101) mit den Elementen (102, 103) verbunden sind, im Querschnitt ausgedehnt sind.

2. Kette nach Anspruch 1, **gekennzeichnet durch** eines oder mehrere der folgenden Merkmale:
(a) wobei jeder der Drahtlitzenleiter (101) als Metalllitzen, -geflecht, -netz (501), -schirm, -lahn (503) oder -schaum (605) ausgebildet ist;
(b) wobei jeder der Drahtlitzenleiter (101) aus Metall gebildet ist, das als Litzen ausgebildet ist, die lose, verseilt oder axial gruppiert sind;
(c) wobei jeder der Drahtlitzenleiter (101) aus Metall gebildet ist, das als Litzen ausgebildet ist, die zur Verdichtung nahe dem Element zusammengepresst und zur Ausdehnung entfernt von den Elementen (102, 103) auseinandergespreizt sind;
(d) wobei jeder der Drahtlitzenleiter (101) aus Metall gebildet ist, das als Geflecht ausgebildet ist, das in einer runden, ovalen oder flachen Rohrform gewebt ist, und wobei das rohrförmige Geflecht vorzugsweise einen ersten Abschnitt, der angrenzend an die Elemente (102, 103) angeordnet ist, und einen zweiten Abschnitt aufweist, der einen Durchmesser bzw. eine Breite hat, der bzw. die größer als der Durchmesser bzw. die Breite des ersten Abschnitts ist, und weiter entfernt von den Elementen (102, 103) als der erste Abschnitt angeordnet ist;
(e) wobei jeder der Drahtlitzenleiter (101) aus einem Metallnetz oder einem Metallschirm gebildet ist, das bzw. der angrenzend an die Elemente (102, 103) in einer engen Zickzackform und entfernt von den Elementen (102, 103) in einer lockeren Zickzackform gefaltet ist;
(f) wobei jeder der Drahtlitzenleiter (101) aus einem Metallnetz oder -schirm gebildet ist, das bzw. der einen ersten Abschnitt, der angrenzend an das Element (102, 103) angeordnet ist, und einen zweiten Abschnitt aufweist, der lockerer gerollt ist als der erste Abschnitt und sich weiter entfernt von dem Element als der fest gerollte Abschnitt befindet;
(g) wobei die thermoelektrischen Elemente (102, 103) ein Material umfassen, das aus der aus einem Halbleitermaterial, einem dotierten Halbleitermaterial, Blei, Wismut, Antimon, Selen, Tellur, einer Thermotunnelvakuumröhre und einer Kombination daraus bestehenden Gruppe ausgewählt ist;
(h) wobei die thermoelektrischen Elemente (102, 103) abwechselnde N- und P-leitende Elemente (102, 103) umfassen; und
(i) optional ferner mit einer Zugentlastung (106) zum Anbringen und Schützen der thermoelektrischen Elemente (102, 103), wobei (i) die Zugentlastung (106) vorzugsweise aus Leiterplattensubstratmaterial geschnitten oder zusammengesetzt ist, und wobei das Leiterplattensubstratmaterial vorzugsweise aus der aus einem Polyimid, Kapton, Polyester, Nylon, FR-4, Epoxid, Klebstoff und Glasfasern oder Glasfasergewebe bestehenden Gruppe ausgewählt ist, das an die thermoelektrischen Elemente (102, 103) und einen Abschnitt des verdichteten Leiters angrenzt oder diese umgibt; oder (ii) wobei die Zugentlastung (106) Kupfer- oder andere metallische Kontaktstellen zum Befestigen des verdichteten Metalls an der Zugentlastung (106) und am thermoelektrischen Element durch Löten umfasst, und/oder
(j) optional mit einer variablen Stromversorgung, die vom Benutzer gesteuert wird, vorzugsweise einer universellen Stromversorgung mit einer Steuerspannung, die über ein vom Benutzer verstellbares Potentiometer eingestellt wird, und optional mit einem Polaritätsumschalter zur Auswahl von Heizen oder Kühlen, und/oder einem Thermistor, mit dem die Steuerspannung als Reaktion auf Änderungen der Umgebungstemperatur eingestellt wird.

3. Thermoelektrische Platte, **dadurch gekennzeichnet, dass** sie die Kette nach Anspruch 1 oder 2 umfasst, wobei die thermoelektrischen Elemente (102, 103) in Löcher (302) in einer Isolierplatte (301) ein- und aus diesen ausgefädelt sind, wobei Abschnitte (303) des Metalls in den Löchern (302) in der Platte (301) größtenteils verdichtet sind und Abschnitte außerhalb der Löcher (302) in der Platte (301) größtenteils gestreckt sind, oder Paare thermoelektrischer Elemente (102, 103), die Metall dazwischen aufweisen, von einer Seite einer Isolierplatte (301) aus durch ein Loch geschoben werden, wodurch auf der anderen Seite eine Schlaufe aus gestrecktem oder streckbarem Metall freiliegt und die Elemente (102, 103) innerhalb der Platte (301) gehalten werden, wobei die Platte (301) vorzugsweise aus einem Material besteht, das aus der aus Styropor™, natürlichem Stoff, synthetischem Stoff, Naturschwamm, synthetischem Schwamm, Polyurethan, Glasfaser, Schaumglas, Gebäudedämmmaterial, Holz, Papier, Baumwolle, Watte, Rohrumwicklungsdämmstoff, Deckenplattenmaterial, Memory-Schaum und einem Polstermaterial bestehenden Gruppe ausgewählt ist.

4. Thermoelektrische Vorrichtung, **dadurch gekennzeichnet, dass** sie mehrere thermoelektrische Platten nach Anspruch 3 umfasst, die in aufsteigender oder absteigender thermischer Reihenfolge aufeinandergestapelt sind, um größere Temperaturunterschiede zu erzielen.

5. Thermoelektrische Vorrichtung nach Anspruch 4, **gekennzeichnet durch** eines oder mehrere der folgenden Merkmale:
(a) wobei die Mehrzahl eine ganze Zahl gleich 2, 3 oder 4 ist;
(b) wobei mehrere Baugruppen aus Kette und Platte (301) miteinander verbunden und auf einer wärmeleitenden Platte oder Gruppe von Platten, vorzugsweise einer Leiterplatte oder Gruppe von Leiterplatten (401), elektrisch isoliert sind;
(c) wobei mehrere Baugruppen aus Kette und Platte (301) miteinander verbunden und auf wärmeleitenden Platten durch ein elektrisches Isoliermaterial, das aus der aus FR4, Kapton und einem Polyimid bestehenden Gruppe ausgewählt ist, elektrisch isoliert sind;
(d) wobei mehrere Baugruppen aus Kette und Platte (301) miteinander verbunden und auf wärmeleitenden Platten (401) durch ein elektrisches Isoliermaterial, das dünn ist oder ein Metallsubstrat mit dünnen Isolierschichten enthält, um eine hohe Wärmeleitung zu ermöglichen, elektrisch isoliert sind, und wobei das dünne elektrische Isoliermaterial vorzugsweise Kapton, ein Polyimid oder ein Oxid eines Metallsubstrats ist und eine Dicke von 10 bis 40 Mikrometern aufweist;
(e) Kupfer- oder andere metallische Kontaktstellen enthält, damit es leichter ist, das Streckmetall außerhalb der gestapelten Platten (301) auf beiden Seiten der Platte bzw. der Platten zu verlöten.

6. Verfahren zur Bildung einer thermoelektrischen Platte nach Anspruch 3, **dadurch gekennzeichnet, dass** es die folgenden Schritte umfasst:
(a) Montieren mehrerer thermoelektrischer Elemente (102, 103) nach Anspruch 1 auf einer Substratplatte, die mit Kontaktstellen mit Zugentlastung (106) strukturiert ist, und Schneiden der mehreren thermoelektrischen Elemente (102, 103) mit Baugruppen mit Zugentlastung (106) von der Substratplatte, oder
(b) Ein- und Ausfädeln mehrerer thermoelektrischer Elemente (102, 103) nach Anspruch 1 in Löcher (302) in einer Isolierplatte (301) und aus diesen heraus, wobei Abschnitte der Drahtlitzenleiter (101) innerhalb der Löcher (302) in der Platte (301) größtenteils verdichtet und Abschnitte außerhalb der Löcher (302) in der Platte (301) größtenteils ausgedehnt sind, wobei dies das Flechten von Bändern aus thermoelektrischen Elementen (102, 103) in einer Form, das Einspritzen eines aushärtbaren Plattenmaterials in die Form, das Aushärtenlassen des Plattenmaterials und das Entformen umfasst.

7. Thermoelektrische Platte nach Anspruch 3, wobei die thermoelektrische Platte so eingerichtet ist, dass sie Elektrizität erzeugt, wenn eine Seite der thermoelektrischen Platte mit Wärme beaufschlagt wird, wobei die Wärme vorzugsweise eine Wärmequelle umfasst, die aus der aus Sonnenlicht, Erdwärme, Abwärme, Körperwärme, tierischer Wärme, Abgaswärme, Motorwärme, Turbinenwärme und Rohrwärme bestehenden Gruppe ausgewählt ist.

8. Gerät zur Speicherung von Sonnenenergie und zur Erzeugung von Elektrizität aus der gespeicherten Energie, **dadurch gekennzeichnet, dass** das Gerät einen transparenten Isolator (903), eine selektive Oberfläche (904), ein Wärmespeichermedium (905), eine verteilte thermoelektrische Platte (902) und einen Kältespeicher umfasst, wobei die verteilte thermoelektrische Platte (902) die wenigstens eine Kette aus thermoelektrischen Elementen (102, 103) nach Anspruch 1 aufweist.

9. Gerät nach Anspruch 8, **gekennzeichnet durch** eines oder mehrere der folgenden Merkmale:
(a) wobei der transparente Isolator (903) aus einem weitgehend durchsichtigen Feststoff und Luft besteht, wobei der Feststoff vorzugsweise aus einem Material gebildet ist, das aus der aus Polyethylen, Acrylglas und Glas bestehenden Gruppe ausgewählt ist, und/oder wobei der Feststoff optional Taschen bildet, die die Luft oder luftleere Bereiche umgeben;
(b) wobei die selektive Oberfläche (904) ein Metalloxid, ein eloxiertes Metall, schwarze Farbe oder eine Kombination daraus umfasst;
(c) wobei die selektive Oberfläche (904) aus der aus Kohlenstoff, Kupfer, Aluminium, Eisen, Stahl und einer Kombination daraus oder einer Legierung davon bestehenden Gruppe ausgewählt ist;
(d) wobei das Wärmespeichermedium (905) ein Material mit hoher Wärmekapazität umfasst, wobei das Material vorzugsweise aus Wasser, Eisen, Asphalt, Glas, Sand, Salz, Kupfer und einer Kombination daraus ausgewählt ist;
(e) wobei das Wärmespeichermedium (905) in einem Behälter eingeschlossen ist, wobei der Behälter vorzugsweise aus Vinyl gebildet ist;
(f) wobei die verteilte thermoelektrische Platte (902) auf einer Seite thermisch mit dem Wärmespeichermedium (905) und auf der anderen Seite thermisch mit dem Kältespeicher verbunden ist, wobei die verteilte thermoelektrische Platte (902) vorzugsweise N- und P-leitende thermoelektrische Elemente (102, 103) aufweist, die elektrisch verbunden sind, und/oder wobei die thermoelektrischen Elemente (102, 103) Thermotunnelvorrichtungen sind;
(g) wobei die verteilte thermoelektrische Platte (902) einen Vakuumhohlraum aufweist;
(h) wobei der Kältespeicher aus der aus dem Erdboden, Erdreich, Sand, einem natürlichen Gewässer und einem künstlichen Gewässer bestehenden Gruppe ausgewählt ist; und
(i) ferner mit einem Wärmeschnittstellenmaterial auf einer oder beiden Seiten der verteilten thermoelektrischen Platte (902).

10. Verwendung einer Kette aus thermoelektrischen Elementen (102, 103) nach Anspruch 1, **dadurch gekennzeichnet, dass** die Kette in ein Sitzpolster, eine Rückenlehne, eine Decke oder einen Deckenabschnitt, ein Kopfkissen, eine Schreibtischunterplatte, eine Raumdeckenplatte, eine Wand oder einen Fußboden oder ein Fenster eines Gebäudes oder Wohnhauses, eine Wand oder eine Tür eines Kühlschranks oder eines Weinkühlers, ein Getränke- oder Krugisolierteil, eine Elektronikgehäusewand, ein tragbares Kleidungsstück oder ein tragbares Uniformteil, einen Helm oder einen Hut oder eine Schutzhelmauskleidung oder ein Fluid enthaltendes Rohr eingearbeitet ist.

11. Verwendung der thermoelektrischen Platte nach Anspruch 3 bei einer Matratze, **dadurch gekennzeichnet, dass** die Platte oben an der Matratze angebracht ist, wobei
(a) die Matratze eine Federkernmatratze ist und ein Abschnitt jedes der Drahtlitzenleiter (101) in dem Hohlraum, der die Federn enthält, freiliegt, und in dem Hohlraum eine erzwungene oder eine natürliche Luftkonvektion vorhanden ist; oder
(b) die Matratze eine Luftmatratze ist und die thermoelektrische Vorrichtung oben an der Luftmatratze angebracht ist und auf einer Seite der Vorrichtung eine thermische Verbindung jedes der Drahtlitzenleiter (101) in den Hohlraum aufweist, der die Luft enthält, und eine Bewegung der Luft in dem Hohlraum vorhanden ist; oder
(c) die Matratze eine Schaumstoffmatratze ist und die thermoelektrische Vorrichtung oben an der dicken Schaumstoffmatratze angebracht ist, bei der sich ein Abschnitt jedes der Drahtlitzenleiter (101) in vertiefte Kanäle erstreckt, die eine natürliche oder erzwungene Luftkonvektion bieten; und optional
(d) wobei die Matratze oben auf einer weiteren Matratze oder einem Sofa oder einer Couch oder einem Sitzunterteil oder einer Rückenlehne als Zubehör verwendet wird; und/oder
(e) wobei die Löcher (302) ferner Schläuche oder Rohre zum zusätzlichen Stützen enthalten; und/oder
(f) ferner mit einem Ventilator oder einer Pumpe zur Bereitstellung einer erzwungenen Luftkonvektion.

12. Verwendung der thermoelektrischen Platte nach Anspruch 3, **dadurch gekennzeichnet, dass** die Platte an der Sitzfläche und/oder an der Rückenlehne eines Stuhls, eines Sofas, einer Ottomane, eines Rollstuhls, eines Kissens oder einer Couch angebracht ist, und wobei die thermoelektrische Platte vorzugsweise hinter oder unter dem bestehenden Stützgitter in der Lehne oder der Sitzfläche angebracht ist, und wobei ein Abschnitt jedes der Drahtlitzenleiter (101) der thermoelektrischen Platte vorzugsweise durch das Gitter ragt, um einen besseren Kontakt mit der Haut oder der Kleidung zu erzielen.

13. Verwendung einer thermoelektrischen Platte nach Anspruch 3 bei einer Matratze, einem Möbel, einer Decke, einem Kissen oder Kleidung, zu der unter anderem ein Hut oder ein Helm gehören, **dadurch gekennzeichnet, dass** auf der Seite der thermoelektrischen Platte (301), die mit der Haut oder der Kleidung in Berührung kommt, über jedem der Drahtlitzenleiter (101) ein Bezugsstoff vorgesehen ist, wobei der Bezugsstoff vorzugsweise aus natürlichem oder synthetischem Gewebe besteht, das dünn und porös ist und eine Luftströmung ermöglicht, oder aus einem Gewebe, einer Folie oder einem Geflecht besteht, das so ausgelegt ist, dass es eine hohe Wärmeleitfähigkeit hat, oder aus einem Material besteht, das seine Phase ändert, wenn es mit menschlicher oder tierischer Haut in Berührung ist, wodurch Wärme bewegt oder abgeführt wird.

## Revendications

1. Chaîne d'éléments thermoélectriques (102, 103), **caractérisée en ce que** les éléments thermoélectriques (102, 103) sont reliés les uns aux autres par des conducteurs à fil toronné (101), chacun des conducteurs à fil toronné (101) étant compacté en section transversale près de l'endroit où les conducteurs à fil toronné (101) se relient aux éléments (102, 103) et étant élargi en section transversale en éloignement de l'endroit où les conducteurs à fil toronné (101) se relient aux éléments (102, 103).

2. Chaîne selon la revendication 1, **caractérisée par** une ou plusieurs des caractéristiques suivantes :
(a) chacun des conducteurs à fil toronné (101) est réalisé sous forme de torons métalliques, de tresse, de maillage (501), d'écran, de guirlande (503) ou de mousse (605) ;
(b) chacun des conducteurs à fil toronné (101) est réalisé en métal formé sous forme de torons libres, cordés ou regroupés axialement ;
(c) chacun des conducteurs à fil toronné (101) est réalisé en métal formé sous forme de torons qui sont comprimés ensemble pour être compactés près de l'élément et qui écartés pour être élargis en éloignement des éléments (102, 103) ;
(d) chacun des conducteurs à fil toronné (101) est réalisé en métal formé sous forme de tresse tissée en une forme tubulaire ronde, ovale ou plate, et la tresse de forme tubulaire présentant de préférence une première partie agencée à proximité des éléments (102, 103) et une deuxième partie qui présente un diamètre ou une largeur supérieur(e) au diamètre ou à la largeur de la première partie et qui est agencée à une plus grande distance des éléments (102, 103) que la première partie ;
(e) chacun des conducteurs à fil toronné (101) est réalisé à partir d'un treillis métallique ou d'un écran métallique qui est plié en une forme en accordéon serré à proximité des éléments (102, 103) et en accordéon lâche à distance des éléments (102, 103) ;
(f) chacun des conducteurs à fil toronné (101) est réalisé à partir d'un treillis ou d'un écran métallique qui présente une première partie agencée à proximité de l'élément (102, 103) et une deuxième partie qui est enroulée de façon plus lâche que la première partie et qui est agencée à une plus grande distance de l'élément que la partie enroulée de manière serrée ;
(g) les éléments thermoélectriques (102, 103) sont constitués par un matériau choisi dans le groupe composé d'un matériau semi-conducteur, d'un matériau semi-conducteur dopé, de plomb, de bismuth, d'antimoine, de sélénium, de tellure, d'un tube à vide à effet de thermo-tunnel et d'une combinaison de ces éléments ;
(h) les éléments thermoélectriques (102, 103) comprennent des éléments (102, 103) de type N et P alternés ; et
(i) comprenant facultativement en outre un moyen de décharge de traction (106) pour le montage et la protection des éléments thermoélectriques (102, 103), (i) le moyen de décharge de traction (106) étant de préférence découpé ou assemblé à partir d'un matériau de substrat de carte de circuit imprimé, et le matériau de substrat de carte de circuit imprimé étant de préférence choisi dans le groupe composé d'un polyimide, de Kapton, de polyester, de nylon, de FR-4, d'époxy, de colle et de fibre de verre ou d'un tissu de fibre de verre à proximité ou entourant les éléments thermoélectriques (102, 103) et une partie du conducteur compacté ; ou (ii) le moyen de décharge de traction (106) comprenant des plots de cuivre ou d'autres plots métalliques pour fixer par brasage le métal compacté au moyen de décharge de traction (106) et à l'élément thermoélectrique, et/ou
(j) présentant facultativement une alimentation en courant variable qui est commandée par l'utilisateur, de préférence une alimentation en courant universelle avec une tension de commande fixée par un potentiomètre réglable par l'utilisateur, et présentant facultativement un commutateur de polarité pour sélectionner le chauffage ou le refroidissement, et/ou une thermistance qui règle la tension de commande en réponse à des changements de la température ambiante.

3. Panneau thermoélectrique, **caractérisé en ce qu'**il comprend la chaîne selon la revendication 1 ou 2, les éléments thermoélectriques (102, 103) étant tissés dans et hors des trous (302) dans un panneau isolant (301) dans lequel des parties (303) du métal à l'intérieur des trous (302) dans le panneau (301) sont en majeure partie compactées et des parties à l'extérieur des trous (302) dans le panneau (301) sont en majeure partie élargies, ou des paires d'éléments thermoélectriques (102, 103) présentant du métal entre eux étant poussées à travers un trou d'un côté d'un panneau isolant (301) exposant une boucle de métal déployé ou apte à être déployé de l'autre côté et retenant les éléments (102, 103) à l'intérieur du panneau (301), le panneau (301) étant de préférence réalisé en un matériau choisi dans le groupe composé de Styrofoam™, de tissu naturel, de tissu synthétique, de matériau spongieux naturel, de matériau spongieux synthétique, de polyuréthane, de fibre de verre, de verre mousse, de matériau d'isolation de construction, de bois, de papier, de coton, de rembourrage, d'isolation d'emballage de tuyaux, de matériau de dalle de plafond, de mousse à mémoire et d'un matériau d'amortissement.

4. Dispositif thermoélectrique, **caractérisé en ce qu'**il comprend une pluralité de panneaux thermoélectriques selon la revendication 3 qui sont empilés dans un ordre thermique ascendant ou descendant afin d'obtenir des différences de température plus importantes.

5. Dispositif thermoélectrique selon la revendication 4, **caractérisé par** une ou plusieurs des caractéristiques suivantes :
(a) la pluralité est un nombre entier égal à 2, 3 ou 4 ;
(b) plusieurs ensembles de chaînes ou de panneaux (301) sont reliés les uns aux autres et sont électriquement isolés sur une carte ou un groupe de cartes thermiquement conductrices, de préférence une carte de circuit imprimé ou un groupe de cartes de circuit imprimé (401) ;
(c) plusieurs ensembles de chaînes ou de panneaux (301) sont reliés les uns aux autres et sont électriquement isolés sur des cartes thermiquement conductrices par un matériau d'isolation électrique choisi dans le groupe composé de FR4, de Kapton et d'un polyimide ;
(d) plusieurs ensembles de chaînes ou de panneaux (301) sont reliés les uns aux autres et sont électriquement isolés sur des cartes thermiquement conductrices (401) par un matériau d'isolation électrique qui est mince ou qui contient un substrat métallique avec des couches d'isolation minces pour permettre une conduction thermique élevée, et le matériau d'isolation électrique mince étant de préférence du Kapton, un polyimide ou un oxyde d'un substrat métallique et présentant une épaisseur de 10 à 40 microns ;
(e) contenant des plots de cuivre ou d'autres plots métalliques pour faciliter le brasage du métal déployé à l'extérieur des panneaux empilés (301) de chaque côté de la carte ou des cartes.

6. Procédé de réalisation d'un panneau thermoélectrique selon la revendication 3, **caractérisé en ce qu'**il comporte les étapes suivantes :
(a) assemblage d'une pluralité d'éléments thermoélectriques (102, 103) selon la revendication 1 sur une carte de substrat structurée avec des plots d'un moyen de décharge de traction (106), et découpe des plusieurs ensembles d'éléments thermoélectriques (102, 103) avec un moyen de décharge de traction (106) à partir de la carte de substrat, ou
(b) tissage d'une pluralité d'éléments thermoélectriques (102, 103) selon la revendication 1 dans et hors des trous (302) d'un panneau isolant (301), des parties des conducteurs à fil toronné (101) à l'intérieur des trous (302) dans le panneau (301) étant en majeure partie compactées et des parties à l'extérieur des trous (302) dans le panneau (301) étant en majeure partie élargies, ce qui comprend le tissage de chaînes d'éléments électrothermiques (102, 103) dans un moule, l'injection d'un matériau de panneau durcissable dans le moule, laisser le matériau de panneau durcir, et le retrait du moule.

7. Panneau thermoélectrique selon la revendication 3, le panneau thermoélectrique étant réalisé de manière à produire de l'électricité lorsque de la chaleur est appliquée chaleur d'un côté du panneau thermoélectrique, la chaleur comprenant de préférence une source de chaleur choisie dans le groupe composé de lumière solaire, de chaleur géothermique, de chaleur perdue, de chaleur corporelle, de chaleur animale, de chaleur de gaz d'échappement, de chaleur de moteur, de chaleur de turbine et de chaleur de tuyau.

8. Appareil pour le stockage d'énergie solaire et la génération d'électricité à partir de l'énergie stockée, **caractérisé en ce que** l'appareil comprend un isolant transparent (903), une surface sélective (904), un moyen de stockage de chaleur (905), un panneau thermoélectrique distribué (902) et un réservoir de froid, le panneau thermoélectrique distribué (902) présentant ladite au moins une chaîne d'éléments électriques (102, 103) selon la revendication 1.

9. Appareil selon la revendication 8, **caractérisé par** une ou plusieurs des caractéristiques suivantes :
(a) l'isolant transparent (903) est composé d'un solide en majeure partie clair et d'air ; le solide étant de préférence réalisé en un matériau choisi dans le groupe composé de polyéthylène, de plexiglas et de verre, et/ou le solide formant facultativement des poches qui entourent l'air ou des zones évacuées ;
(b) la surface sélective (904) comprend un oxyde métallique, un métal anodisé, une peinture noire et une combinaison de ceux-ci ;
(c) la surface sélective (904) est choisie dans le groupe composé de carbone, de cuivre, d'aluminium, de fer, d'acier et d'une combinaison ou d'un alliage de ceux-ci ;
(d) le moyen de stockage de chaleur (905) comprend un matériau à capacité thermique élevée, le matériau étant de préférence choisi parmi l'eau, le fer, l'asphalte, le verre, le sable, le sel, le cuivre et une combinaison de ceux-ci ;
(e) le moyen de stockage de chaleur (905) est renfermé dans un réceptacle, le réceptacle étant de préférence réalisé en vinyle ;
(f) le panneau thermoélectrique distribué (902) est thermiquement relié au moyen de stockage de chaleur (905) d'un côté et est thermiquement relié au réservoir de froid de l'autre côté, le panneau thermoélectrique distribué (902) présentant de préférence des éléments thermoélectriques (102, 103) du type n et du type p électriquement reliés, et/ou les éléments thermoélectriques (102, 103) étant des dispositifs à effet de thermo-tunnel ;
(g) le panneau thermoélectrique distribué (902) présente une cavité à vide ;
(h) le réservoir de froid est choisi dans le groupe composé du sol, de terre, de sable, d'un corps d'eau naturel et d'un corps d'eau artificiel ; et
(i) comprenant en outre un matériau d'interface thermique d'un ou des deux côtés du panneau thermoélectrique distribué (902).

10. Utilisation d'une chaîne d'éléments thermoélectriques (102, 103) selon la revendication 1, **caractérisée en ce que** la chaîne est incorporée dans un coussin de siège, un dossier de siège, une couverture ou une partie de couverture, un oreiller, un panneau de sous-bureau, une dalle de plafond, une paroi ou un plancher ou une fenêtre de bâtiment ou de logement, une paroi ou une porte de réfrigérateur ou de réfrigérateur à vin, un isolant pour boisson ou pichet, une paroi d'enceinte électronique, une pièce de vêtement portable ou d'uniforme, un casque ou un chapeau ou une doublure de casque de protection ou un tuyau contenant du fluide.

11. Utilisation du panneau thermoélectrique selon la revendication 3 dans un matelas, **caractérisée en ce que** le panneau est agencé sur le dessus du matelas,
(a) le matelas étant un matelas à ressorts, et une partie de chacun des conducteurs à fil toronné (101) étant exposée dans la cavité contenant les ressorts, et une convection d'air forcée ou naturelle étant disponible dans la cavité ; ou
(b) le matelas étant un matelas pneumatique, et le dispositif thermoélectrique étant agencé sur le dessus du matelas pneumatique et présentant une connexion thermique de chacun des conducteurs à fil toronné (101) d'un côté du dispositif dans la cavité contenant l'air, et le mouvement de l'air étant disponible dans la cavité ; ou
(c) le matelas étant un matelas en mousse et le dispositif thermoélectrique étant monté sur le dessus du matelas en mousse épaisse dans lequel une partie de chacun des conducteurs à fil toronné (101) s'étend dans des canaux creux qui fournissent une convection d'air naturelle ou forcée ; et facultativement
(d) le matelas étant utilisé sur le dessus d'un autre matelas ou d'un sofa ou d'un canapé ou d'une assise de siège ou d'un dossier de siège en tant qu'accessoire ; et/ou
(e) les trous (302) contenant également des conduites ou des tubes pour un support supplémentaire ; et/ou
(f) comprenant en outre une soufflante ou une pompe pour fournir une convection d'air forcée.

12. Utilisation du panneau thermoélectrique selon la revendication 3, **caractérisée en ce que** le panneau est monté sur une assise de siège ou sur un dossier de siège, ou sur les deux, d'une chaise, d'un sofa, d'une ottomane, d'un fauteuil roulant, d'un oreiller ou d'un canapé, et le panneau thermoélectrique étant de préférence monté derrière ou sous le maillage de support existant dans le dossier ou l'assise, et une partie de chacun des conducteurs à fil toronné (101) du panneau thermoélectrique faisant de préférence saillie à travers le maillage pour obtenir un meilleur contact avec la peau ou le vêtement.

13. Utilisation d'un panneau thermoélectrique selon la revendication 3 dans un matelas, un meuble, une couverture, un oreiller ou un vêtement comprenant, sans y être limité, un chapeau ou un casque, **caractérisée en ce qu'**une étoffe de revêtement est prévue sur chacun des conducteurs à fil toronné (101) du côté du panneau thermoélectrique (301) qui contacte la peau ou le vêtement, l'étoffe de revêtement étant de préférence composée d'un tissu naturel ou synthétique qui est mince et poreux et permet un écoulement d'air, ou étant composée d'un tissu, d'un film ou d'un maillage qui est destiné à présenter une conductivité thermique élevée, ou étant composée d'un matériau qui change de phase lorsqu'il est en contact avec la peau d'un humain ou d'un animal, déplaçant ou évacuant ainsi de la chaleur.
